# EUROPÄISCHE PATENTANMELDUNG

(11) **EP 0 285 947 A2**
(43) Veröffentlichungstag der Anmeldung: **12.10.1988**
(21) Anmeldenummer: 88104948.0
(22) Anmeldetag: 28.03.1988
(51) Int. Cl.: C07D 231/14, C07D 231/12, A01N 43/56, C07D 261/08, C07C 109/087, C07C 109/14

(54) **3-Halogenalkyl-1-aryl-pyrazole**

(30) Priorität: 10.04.1987 DE 3712204
(71) Anmelder: BAYER AG, 51368 Leverkusen (DE)
(72) Erfinder: Ooms, Pieter, Dr., D-4150 Krefeld 1 (DE); Klausener, Alexander, Dr., D-5190 Stolberg-Breinig (DE); Baasner, Bernd, Dr., D-5090 Leverkusen 3 (DE); Becker, Benedikt, Dr., D-4020 Mettmann (DE); Behrenz, Wolfgang, Dr., D-5063 Overath (DE); Homeyer, Bernhard, Dr., D-5090 Leverkusen 3 (DE)

(57) **Zusammenfassung**

Es werden neue 3-Halogenalkyl-1-aryl-pyrazole der allgemeinen Formel (I) 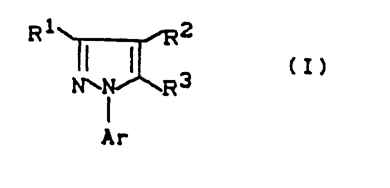 bereitgestellt,
in welcher
R¹ für Halogenalkyl steht,
R² für Cyano, für Hydroxycarbonyl, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkylcarbonyl, Halogenalkylcarbonyl oder Cycloalkylcarbonyl steht,
R³ für Wasserstoff, Alkyl, Halogenalkyl oder Cycloalkyl steht und
Ar für substituiertes Phenyl mit Ausnehme des 2,4-Dinitrophenylrestes steht.

Die neuen Verbindungen (I) besitzen eine stark ausgeprägte Wirksamkeit gegen tierische Schädlingen, insbesondere gegen Insekten.

## Beschreibung

Die Erfindung betrifft neue 3-Halogenalkyl-1-aryl-pyrazole, mehrere Verfahren zu ihrer Herstellung und ihre Verwendung als Schädlingsbekämpfungsmittel.

Es ist bereits bekannt, daß bestimmte 5-Amino-1-aryl-pyrazole, wie beispielsweise das 4-Cyano-5-propionamido-1-(2,4,6-trichlorphenyl)-pyrazol herbizide Wirkung besitzen (vergl. z. B. DE-OS 32 26 513).

Weiterhin sind bestimmte 3-Halogenalkyl-1-aryl-pyrazole, wie beispielsweise das 4-Methoxycarbonyl-1-phenyl-3-trifluormethyl-pyrazol oder das 4-Acetyl-5-methyl-1-(2,4-dinitrophenyl)-3-trichlormethyl-pyrazol oder das 4-Acetyl-5-methyl-1-phenyl-3-trifluormethyl-pyrazol oder das 4-Methoxycarbonyl-5-methyl-1-phenyl-3-trifluormethyl-pyrazol bekannt (vergl. z. B. Bull. chem. Soc. Japan. 59, 2631, [1986]; Chemistry Letters 1982, 543 - 546; J. Heterocycl. Chem. 22, 565 - 568 [1985]; Ind. J. Chem., Sect. B, 19B, 217 - 218 [1980]).

Über eine Wirksamkeit dieser vorbekannten Verbindungen gegen Schädlinge ist bisher nichts bekannt.

Es wurden neue 3-Halogenalkyl-1-aryl-pyrazole der allgemeinen Formel (I), 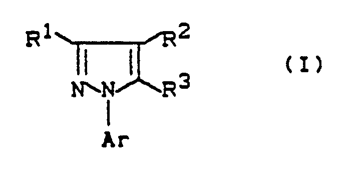 in welcher
R¹ für Halogenalkyl steht.
R² für Cyano, für Hydroxycarbonyl, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkyl-carbonyl, Halogenalkylcarbonyl oder Cycloalkylcarbonyl steht,
R³ für Wasserstoff, Alkyl, Halogenalkyl oder Cycloalkyl steht und
Ar für substituiertes Phenyl mit Ausnahme des 2,4-Dinitrophenylrestes steht,
gefunden.

Weiterhin wurde gefunden, daß man die neuen 3-Halogenalkyl-1-aryl-pyrazole der allgemeinen Formel (I), 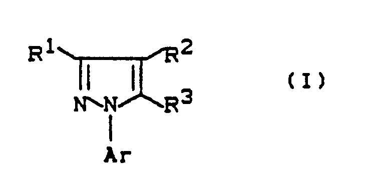 in welcher
R¹ für Halogenalkyl steht,
R² für Cyano, für Hydroxycarbonyl, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkylcarbonyl, Halogenalkylcarbonyl oder Cycloalkylcarbonyl steht,
R³ für Wasserstoff, Alkyl, Halogenalkyl oder Cycloalkyl steht und
Ar für substituiertes Phenyl mit Ausnahme des 2,4-Dinitrophenylrestes steht,
nach den im Folgenden beschriebenen Verfahren erhält.
(a) Man erhält 3-Halogenalkyl-1-aryl-pyrazole der Formel (Ia), 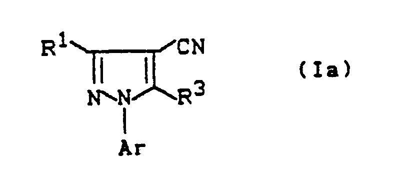 in welcher
   R¹, R³ und Ar die oben angegebene Bedeutung haben,
   wenn man N-Aryl-hydrazidhalogenide der Formel (II),
   R¹- 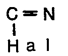 -NH-Ar (II)

   in welcher
   Hal für Halogen steht und
   R¹ und Ar die oben angegebene Bedeutung haben,
   mit Isoxazolen der Formel (III), 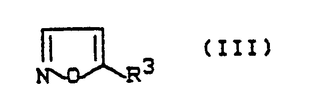 in welcher
   R³ die oben angegebene Bedeutung hat,
   gegebenenfalls in Gegenwart eines Verdünnungsmittels und gegebenenfalls in Gegenwart eines Säurebindemittels umsetzt;
(b) man erhält 3-Halogenalkyl-1-aryl-pyrazole der Formel (Ib), 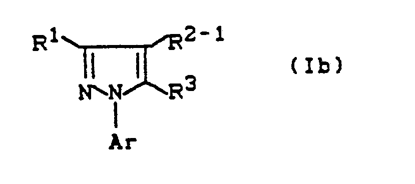 in welcher
   R²⁻¹ für Cyano, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkylcarbonyl, Halogenalkylcarbonyl oder Cycloalkylcarbonyl steht und
   R¹, R³ und Ar die oben angegebene Bedeutung haben,
   wenn man N-Aryl-hydrazidhalogenide der Formel (II),
   R¹- 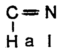 -NH-Ar (II)

   in welcher
   Hal für Halogen steht und
   R¹ und Ar die oben angegebene Bedeutung haben,
   entweder
   (α) mit Carbonylverbindungen der Formel (IV),

   R²⁻¹-CH₂- 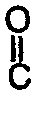 -R³ (IV)
   in welcher
   R²⁻¹ und R³ die oben angegebene Bedeutung haben,
   oder
   (β) mit Acrylnitrilderivaten der Formel (V), 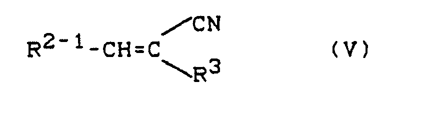 in welcher
   R²⁻¹ und R³ die oben angegebene Bedeutungen haben,
   oder
   (γ) mit Alkinen der Formel (VI),
   R²⁻¹-C≡C-R³ (VI)
   in welcher
   R²⁻¹ und R³ die oben angegebene Bedeutung haben,
   jeweils gegebenenfalls in Gegenwart eines Verdünnungsmittels und gegebenenfalls in Gegenwart eines Säurebindemittels umsetzt;
(c) man erhält 3-Halogenalkyl-1-aryl-pyrazole der Formel (Ic), 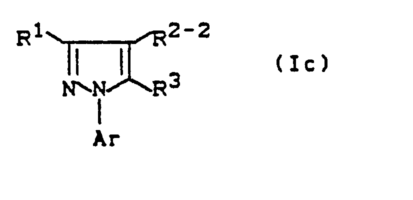 in welcher
   R¹, R³ und Ar die oben angegebene Bedeutung haben
   und
   R²⁻² für Hydroxycarbonyl oder Carbamoyl steht,
   wenn man die mit Hilfe der Verfahren (a) oder (b) erhältlichen 3-Halogenalkyl-1-aryl-pyrazole der Formel (Id) 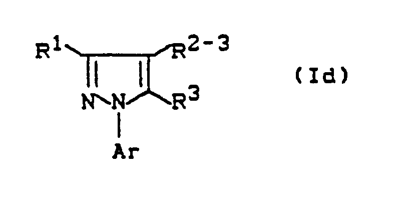 in welcher
   R¹, R³ und Ar die oben angegebene Bedeutung haben und
   R²⁻³ für Cyano, Alkoxycarbonyl, Alkenyloxycarbonyl oder Alkinyloxy steht,
   mit Säuren gegebenenfalls in Gegenwart eines Verdünnungsmittels am Substituenten in 4-Position des Pyrazolringes verseift.

Schließlich wurde gefunden, daß die neuen 3-Halogenalkyl-1-aryl-pyrazole der Formel (I) insektizide Wirkung besitzen.

Überraschenderweise zeigen die erfindungsgemäßen 3-Halogenalkyl-1-aryl-pyrazole der allgemeinen Formel (I) eine bessere insektizide Wirksamkeit, als die aus dem Stand der Technik bekannten 5-Amino-1-aryl-pyrazole, wie beispielswiese das 4-Cyano-5-propionamido-1-(2,4,6-trichlorphenyl)-pyrazol, welche chemisch naheliegende Verbindungen sind.

Die erfindungsgemäßen 3-Halogenalkyl-1-aryl-pyrazole sind durch die Formel (I) allgemein definiert. Bevorzugt sind Verbindungen der Formel (I), bei welchen
R¹ für geradkettiges oder verzweigtes Halogenalkyl mit 1 bis 4 Kohlenstoffatomen und 1 bis 9 gleichen oder verschiedenen Halogenatomen steht,
R² für Cyano, für Hydroxycarbonyl, für Carbamoyl, Thiocarbamoyl, für jeweils geradkettiges oder verzweigtes Alkoxycarbonyl, N-Alkylcarbamoyl oder N,N-Dialkylcarbamoyl mit jeweils 1 bis 4 Kohlenstoffatomen in den einzelnen Alkylteilen, für jeweils geradkettiges oder verzweigtes Alkenyloxycarbonyl, N-Alkenylcarbamoyl oder N,N-Dialkenylcarbamoyl mit jeweils 3 bis 6 Kohlenstoffatomen in den einzelnen Alkenylteilen, für jeweils geradkettiges oder verzweigtes Alkinyloxycarbonyl, N-Alkinylcarbamoyl oder N,N-Dialkinylcarbamoyl mit jeweils 3 bis 6 Kohlenstoffatomen in den einzelnen Alkinylteilen, oder für gegebenenfalls im Phenylteil einfach oder mehrfach, gleich oder verschieden substituiertes Phenylcarbamoyl steht, wobei als Substituenten in Frage kommen: Halogen, Cyano, Nitro sowie jeweils geradkettiges oder verzweigts Alkyl, Alkoxy, Alkylthio, Halogenalkyl, Halogenalkoxy oder Halogenalkylthio mit jeweils 1 bis 4 Kohlenstoffatomen und im Fall des Halogenalkyl, Halogenalkoxy oder Halogenalkylthio mit 1 bis 9 gleichen oder verschiedenen Halogenatomen; außerdem für jeweils geradkettiges oder verzweigtes Alkylcarbonyl oder Halogenalkylcarbonyl mit jeweils 1 bis 5 Kohlenstoffatomen und im Fall des Halogenalkylcarbonyl mit 1 bis 9 gleichen oder verschiedenen Halogenatomen oder für Cycloalkylcarbonyl mit 4 bis 8 Kohlenstoffatomen steht,
R³ für Wasserstoff, für jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen oder für Cycloalkyl mit 3 bis 7 Kohlenstoffatomen steht und
Ar für einfach oder mehrfach, gleich oder verschieden substituiertes Phenyl mit Ausnahme des 2,4-Dinitro phenylrestes steht, wobei als Substituenten infrage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenenfalls 1 bis 9 gleichen oder verschiedenen Halogenatomen.

Besonders bevorzugt sind Verbindungen der Formel (I), bei welchen
R¹ für Trifluormethyl, Dichlorfluormethyl, Difluorchlormethyl, Difluormethyl, Trifluorethyl, Trifluorchlorethyl, Trifluordichlorethyl, Difluordichlorethyl, Tetrafluorethyl, Pentafluorethyl, Heptafluorpropyl oder Nonafluorbutyl steht,
R² für Cyano, für Hydroxycarbonyl, für Carbamoyl, Thiocarbamoyl, für Methoxycarbonyl, Ethoxycarbonyl, N-Methylcarbamoyl, N,N-Dimethylcarbamoyl, N-Ethylcarbamoyl, N,N-Diethylcarbamoyl, für Acetyl, Propionyl, Trifluoracetyl, Dichlorfluoracetyl, Difluorchloracetyl, für Cyclopropylcarbonyl, Cyclohexylcarbonyl oder für im Phenylteil gegebenenfalls ein- bis dreifach gleich oder verschieden durch Fluor, Chlor, Brom, Cyano, Nitro, Methyl, Ethyl, n- oder i-Propyl, Methoxy, Ethoxy, n- oder i-Propoxy, Methylthio, Trifluormethyl, Trifluormethoxy oder Trifluormethylthio substituiertes N-Phenylcarbamoyl steht,
R³ für Wasserstoff, Methyl, Ethyl, n- oder i-Propyl, n-, i-, s- oder t-Butyl, Trifluormethyl, Dichlorfluormethyl, Difluorchlormethyl, Difluormethyl, Trifluorethyl, Tetrafluorethyl, Pentafluorethyl, Trifluorchlorethyl, Trifluordichlorethyl, Difluordichlorethyl, Heptafluorpropyl, Nonafluorbutyl, für Cyclopropyl, Cyclobutyl, Cyclopentyl oder Cyclohexyl steht und
Ar für ein- bis fünffach, gleich oder verschieden substituiertes Phenyl mit Ausnahme des 2,4-Dinitrophenylrestes steht, wobei als Substituenten infrage kommen: Fluor, Chlor, Brom, Iod, Cyano, Nitro, Methyl, Ethyl, n- oder i-Propyl, n-, i-, s- oder t-Butyl, Methoxy, Ethoxy, Methylthio, Methylsulfinyl, Methylsulfonyl, Methoxycarbonyl, Ethoxycarbonyl, Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl, Trifluordichlorethyl oder ein Rest -X-R⁴, wobei X für Sauerstoff, Schwefel, Sulfinyl oder Sulfonyl steht und R⁴ für Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl oder Trifluordichlorethyl steht.

Im einzelnen seien außer den bei den Herstellungsbeispielen genannten Verbindungen die folgenden 3-Halogenalkyl-1-aryl-pyrazole der allgemeinen Formel (I) genannt:

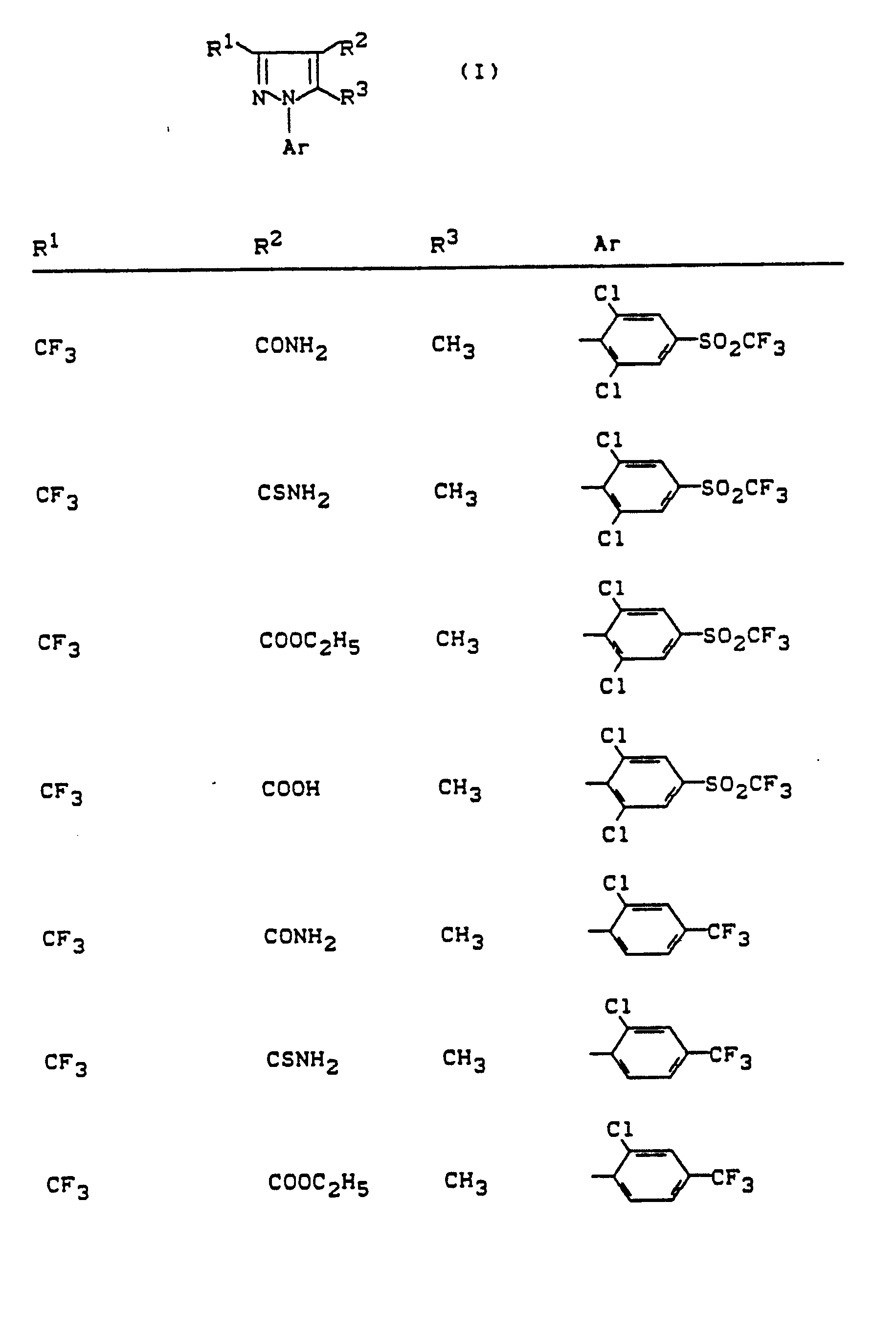

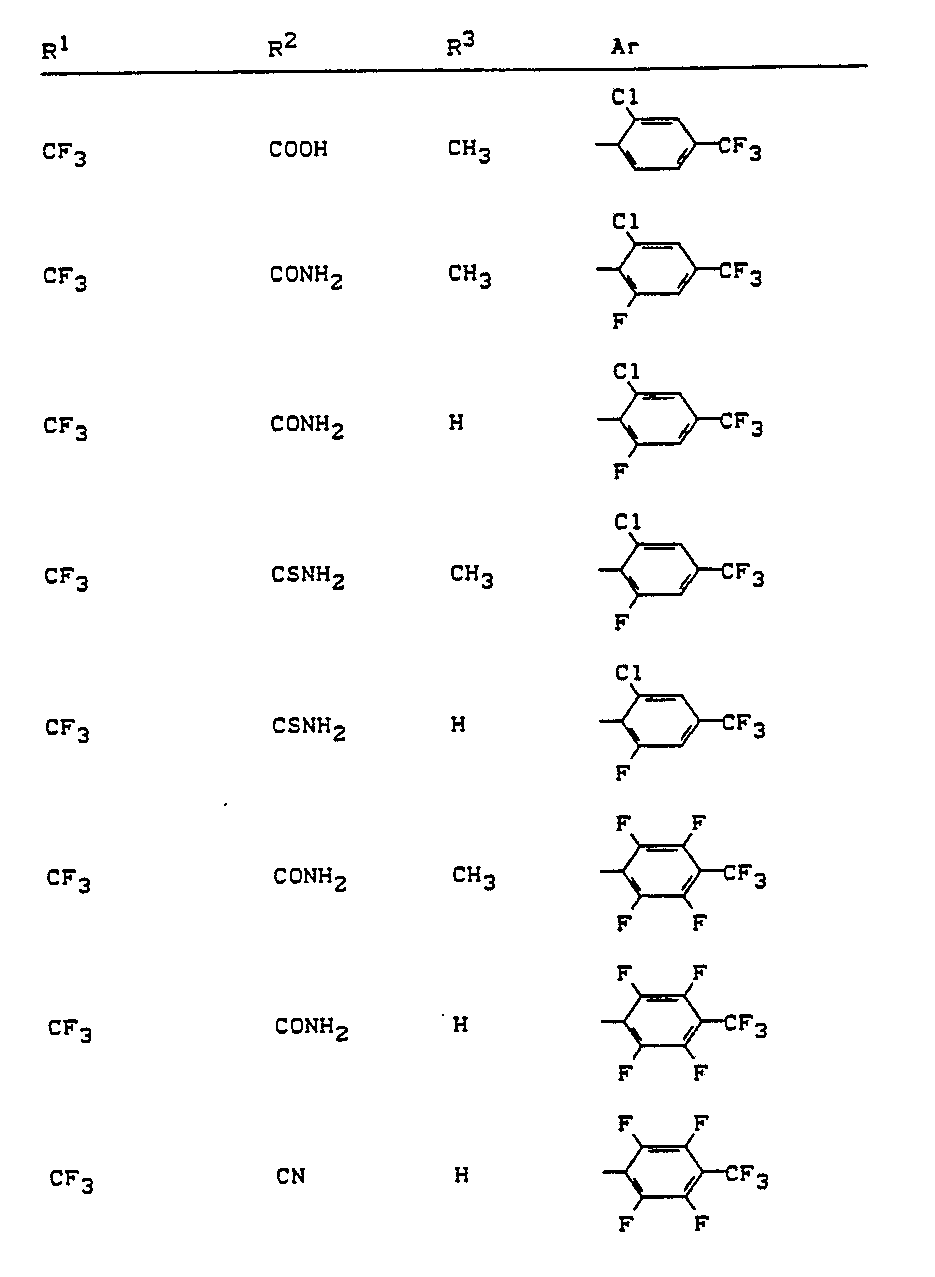

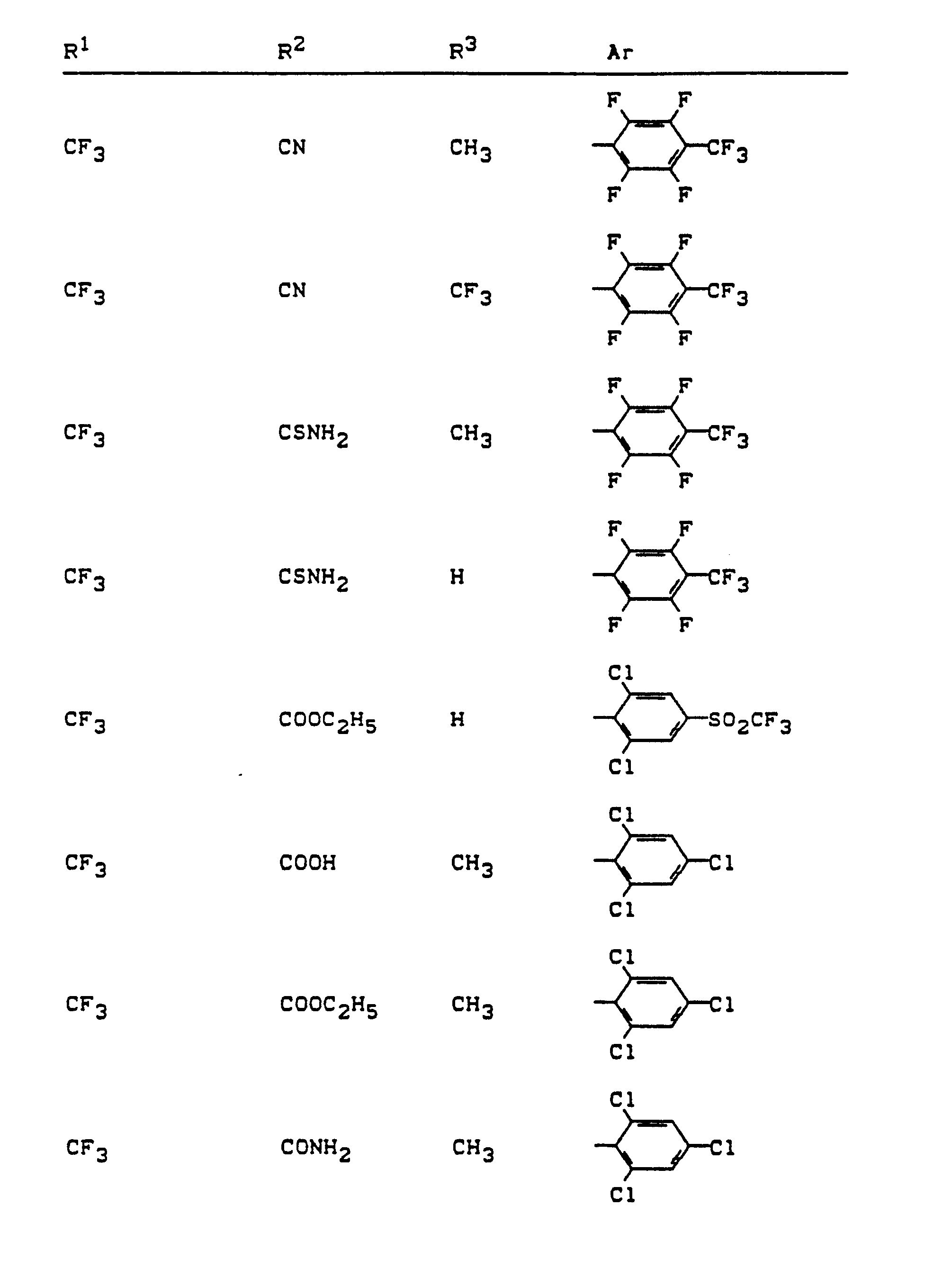

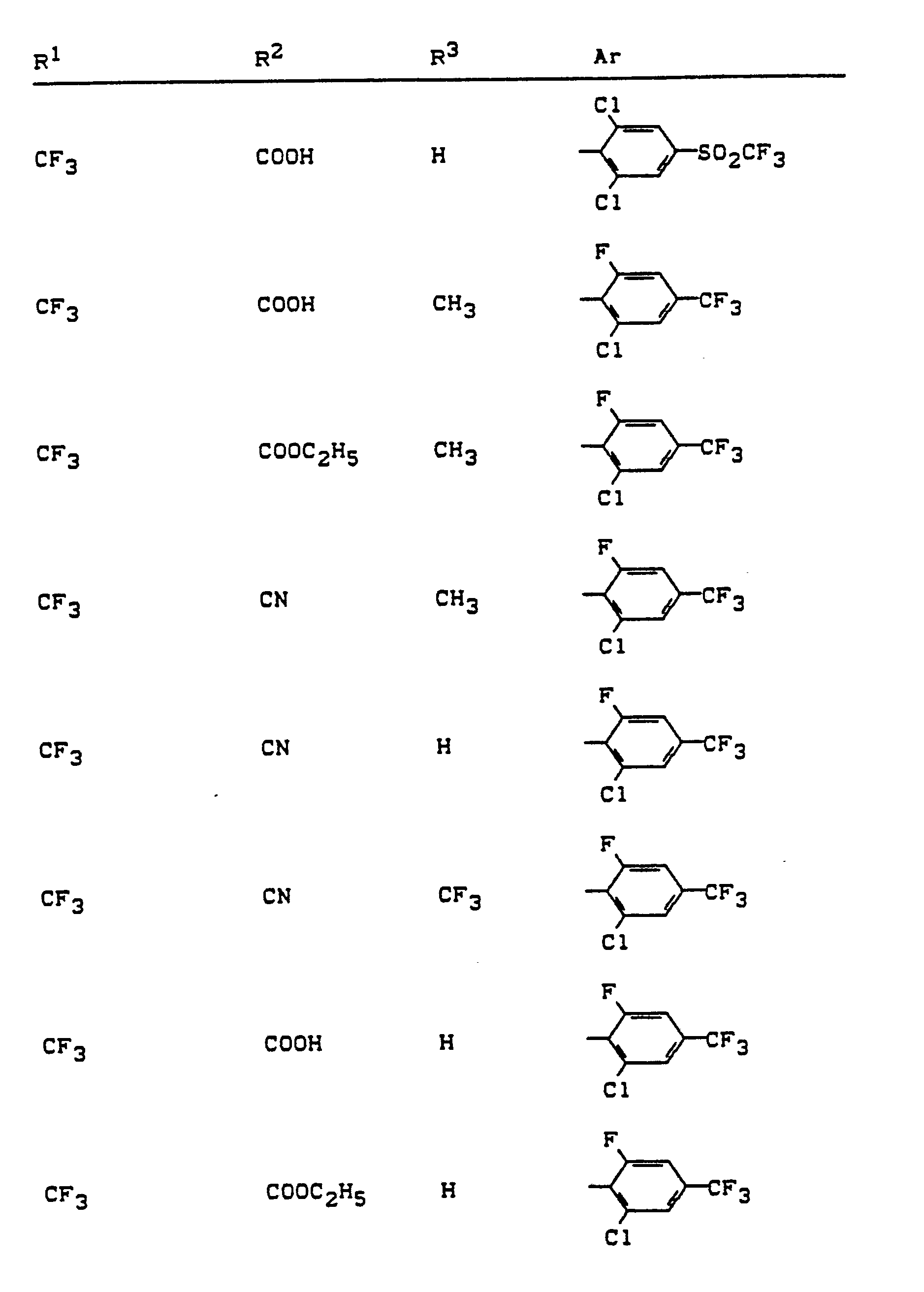

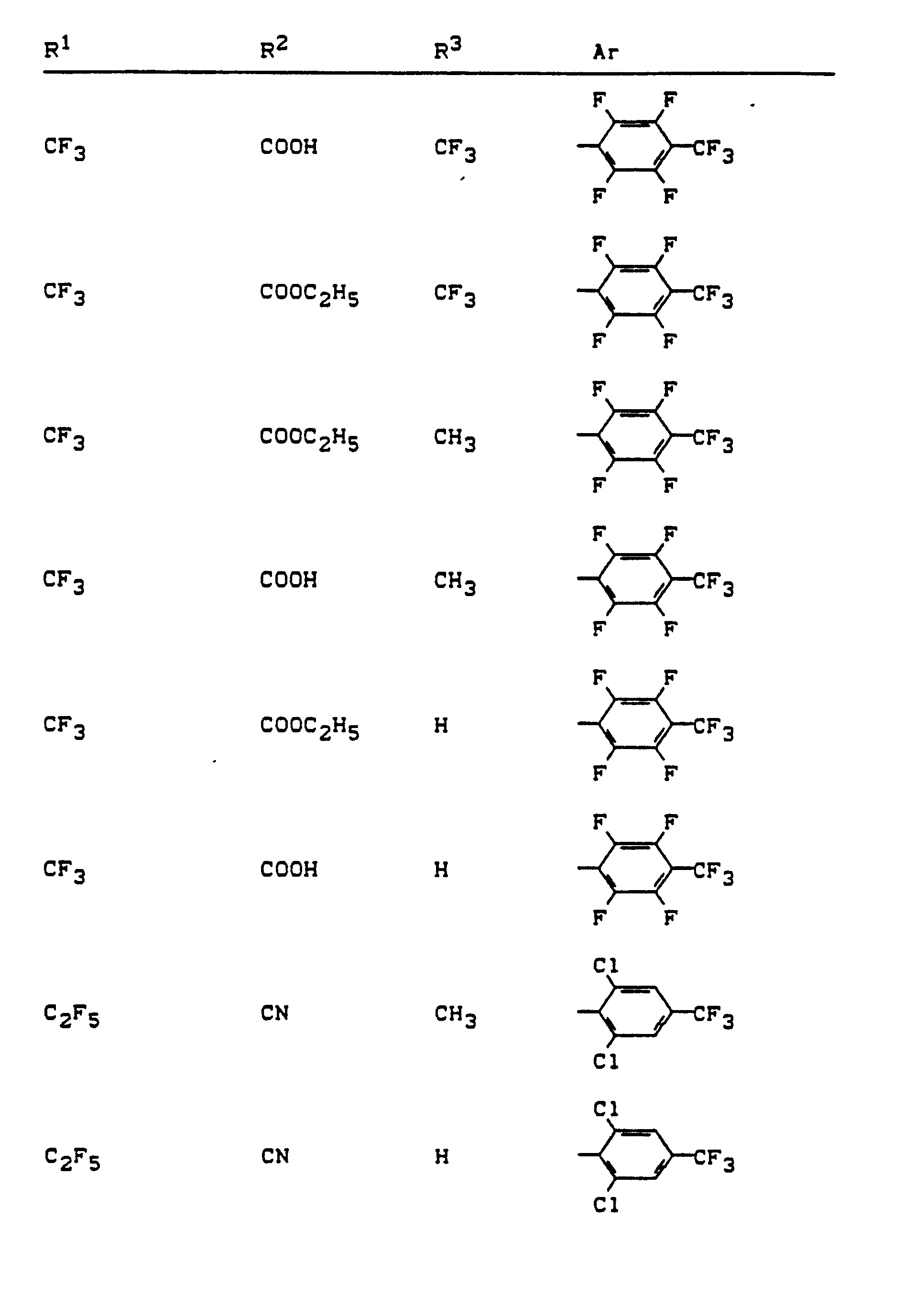

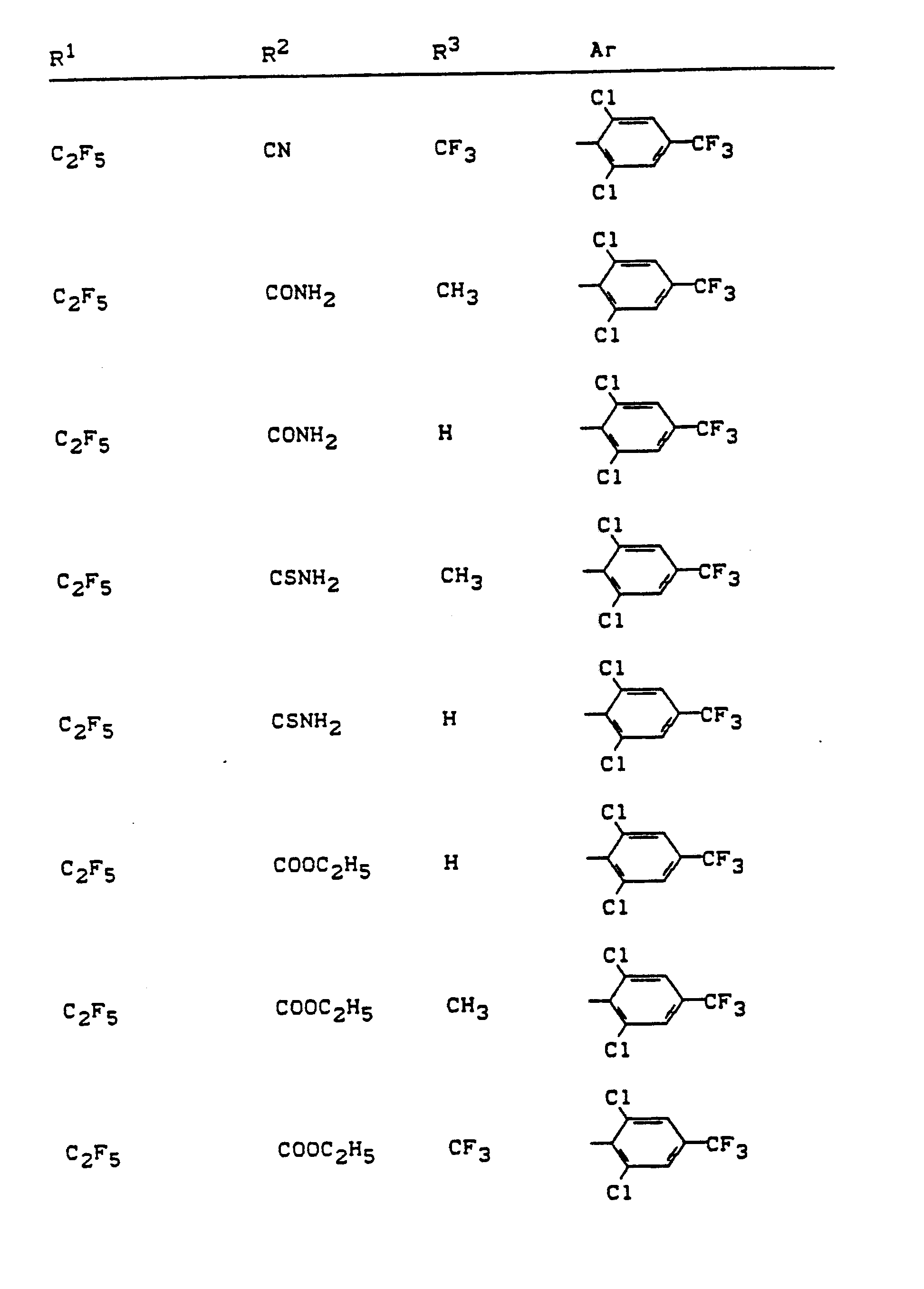

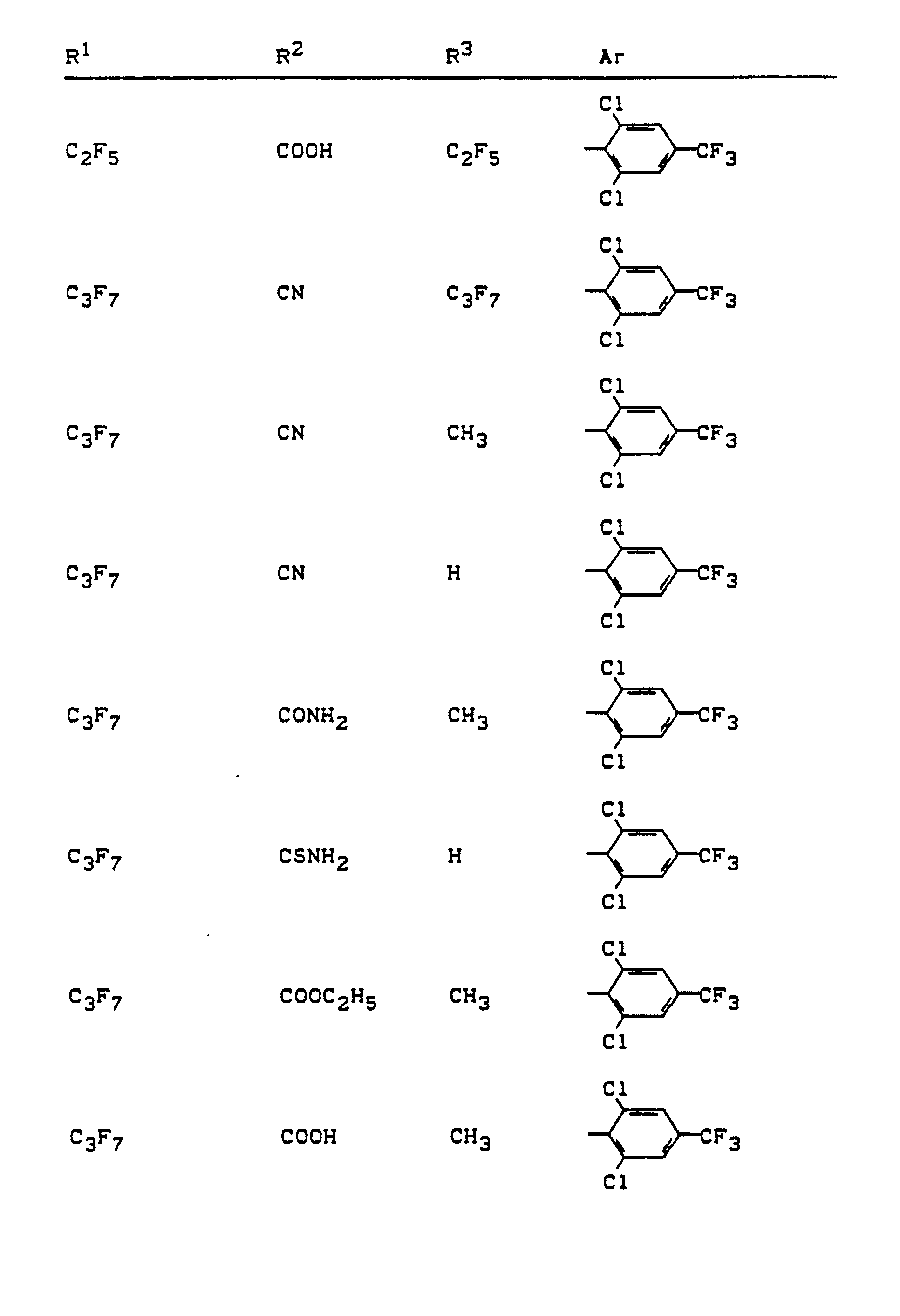

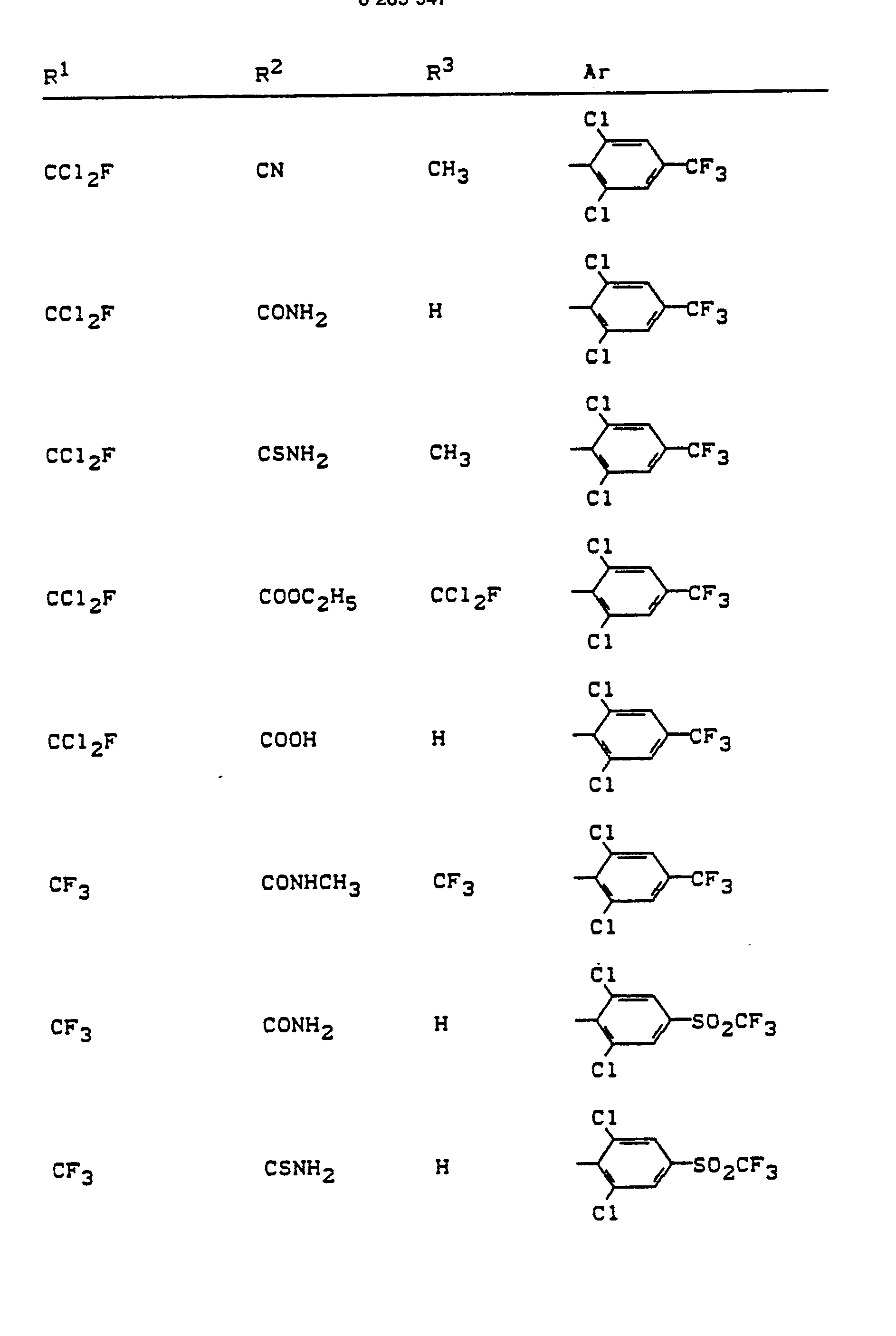

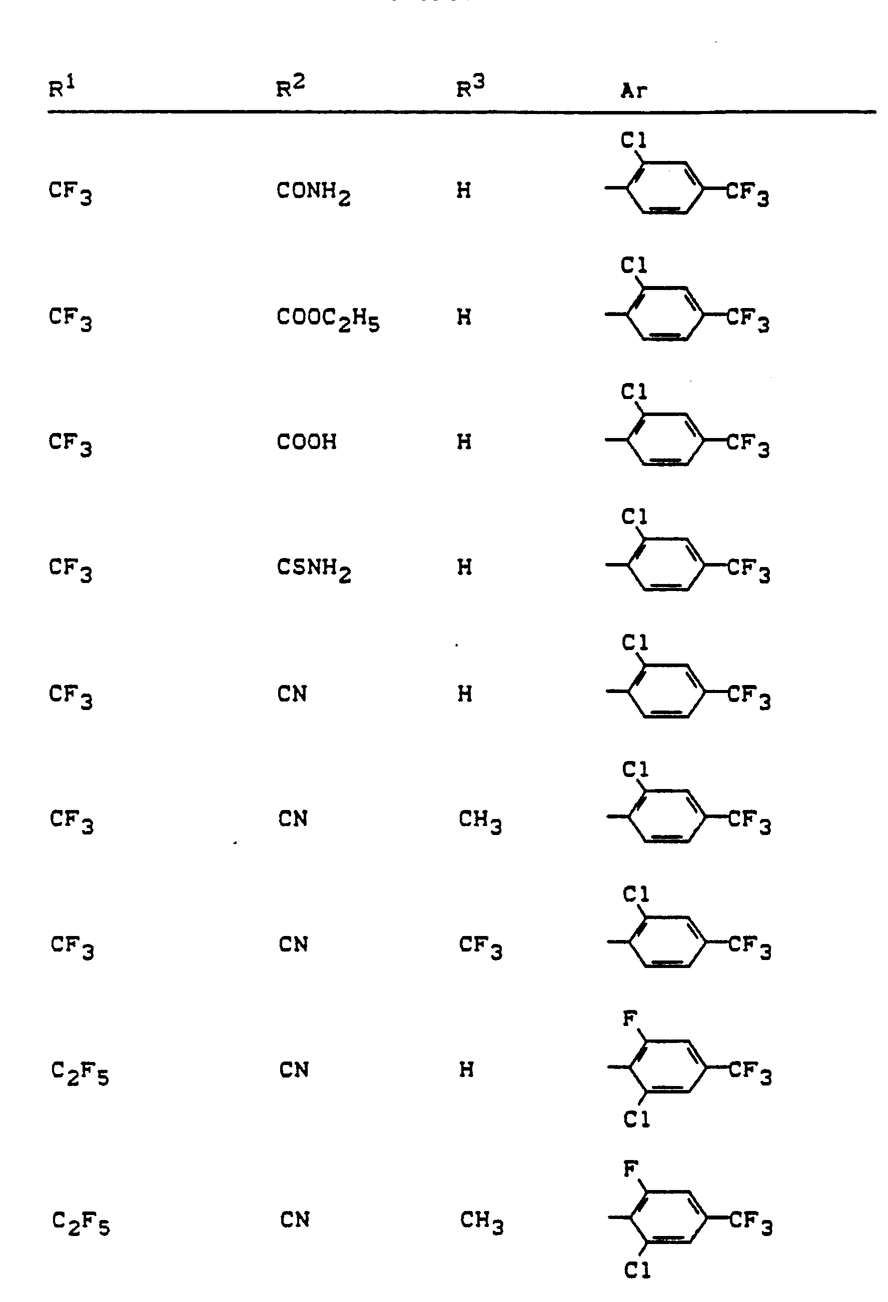

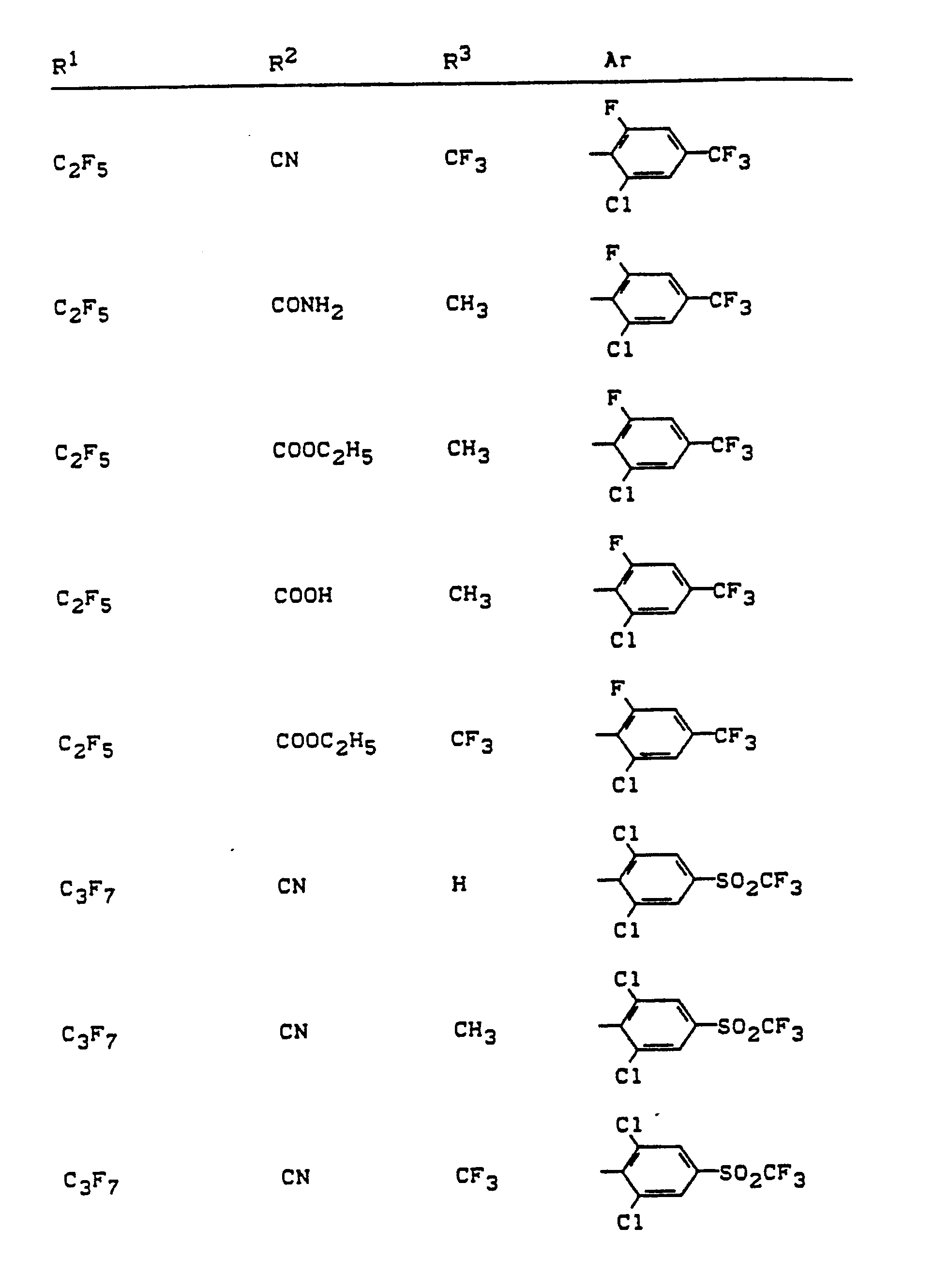

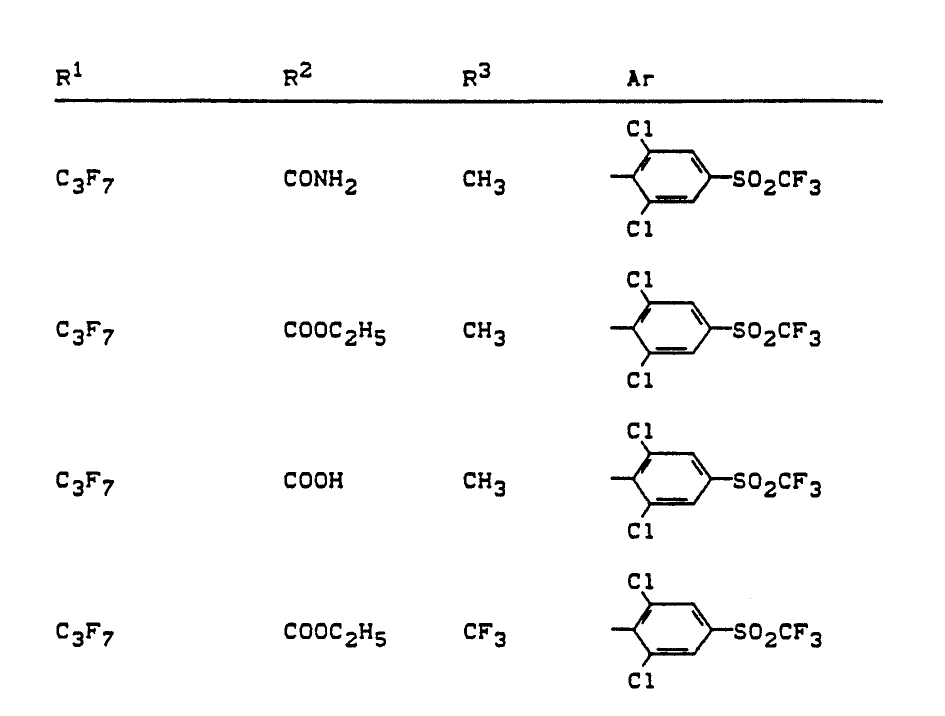

Verwendet man beispielsweise N-(2-Chlor-6-fluor-4-trifluormethylphenyl)-trifluoracethydrazid-bromid und Isoxazol als Ausgangsstoffe, so läßt sich der Reaktionsablauf des erfindungsgemäßen Verfahrens (a) durch das folgende Formelschema darstellen:

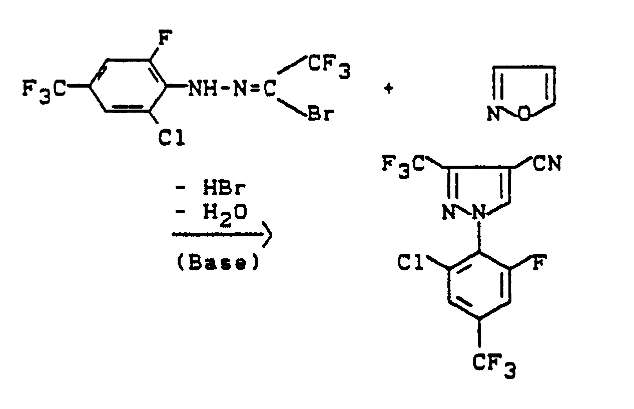

Verwendet man beispielsweise N-(2,4,6-Trichlorphenyl)-trifluoracethydrazid-bromid und Acetessigsäureethylester als Ausgangsstoffe, so läßt sich der Reaktionsablauf des erfindungsgemäßen Verfahrens (b-α) durch das folgende Formelschema darstellen:

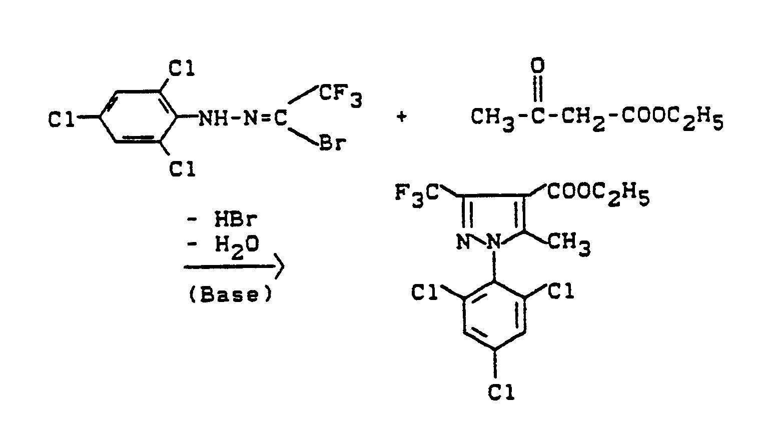

Verwendet man beispielsweise N-(2,3,6-Trichlor-4-trifluormethylphenyl)-trifluoracethydrazid-bromid und Fumarsäuredinitril als Ausgangsstoffe, so läßt sich der Reaktionsablauf des erfindungsgemäßen Verfahrens (b-β) durch das folgende Formelschema darstellen:

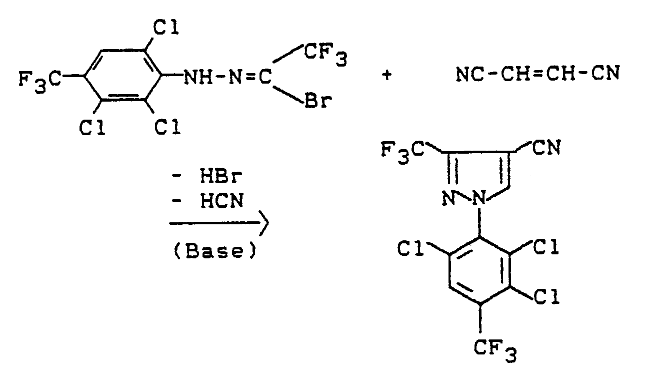

Verwendet man beispielsweise N-(2,6-Dichlor-4-trifluormethylphenyl)-trifluoracethydrazid-bromid und Propionsäuremethylester als Ausgangsstoffe, so läßt sich der Reaktionsablauf des erfindungsgemäßen Verfahrens (b-γ) durch das folgende Formelschema darstellen:

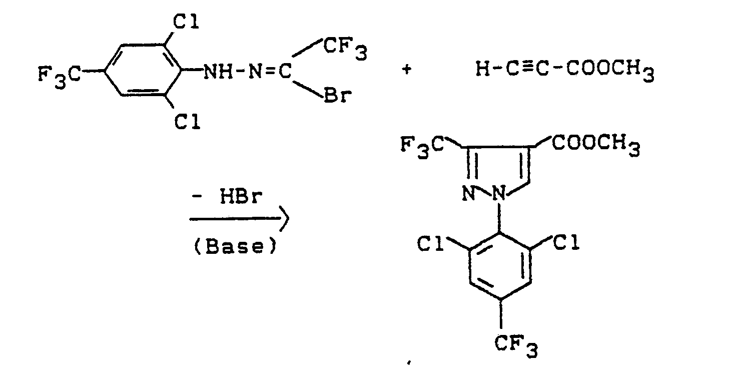

Verwendet man beispielsweise 4-Ethoxycarbonyl-3,5-bis-(trifluormethyl)-1-(2,3,5,6-tetrafluor-4-trifluormethylphenyl)-pyrazol und Schwefelsäure als Ausgangsverbindungen, so läßt sich der Reaktionsablauf des erfindungsgemäßen Verfahrens (c) durch das folgende Formelschema darstellen:

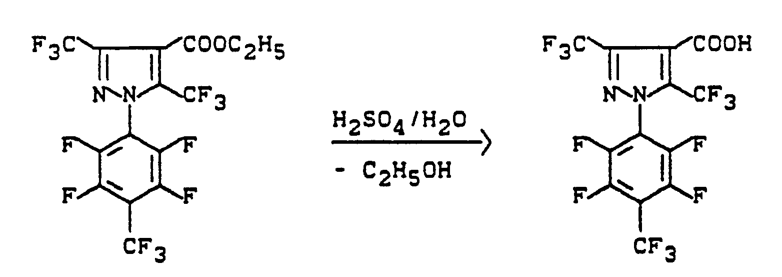

Die zur Durchführung der erfindungsgemäßen Verfahren (a) und (b) als Ausgangsstoffe benötigten N-Arylhydrazidhalogenide sind durch die Formel (II) allgemein definiert. In dieser Formel (II) stehen R¹ und Ar vorzugsweise für diejenigen Reste, die bereits im Zusammenhang mit der Beschreibung der erfindungsgemäßen Stoffe der Formel (I) als bevorzugt für diese Substituenten genannt wurden. Hal steht vorzugsweise für Chlor oder Brom.

Die N-Arylhydrazid-halogenide der Formel (II) sind teilweise bekannt (vergl. z. B. Chem. Lett. 1982, 543; J. Heterocycl. Chem. 22, 565 [1985]; EP 1 019).

Man erhält sie beispielsweise, wenn man Arylhydrazine der Formel (VII),
Ar-NH-NH₂ (VII)
in welcher
Ar die oben angegebene Bedeutung hat,
mit Aldehyden der Formel (VIII) 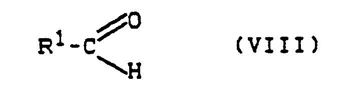 in welcher
R¹ die oben angegebene Bedeutung hat,
oder mit deren Hydraten oder Halbacetalen der Formel (IX), 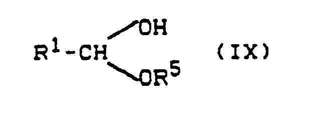 in welcher
R¹ die oben angegebene Bedeutung hat und
R⁵ für Wasserstoff oder Alkyl steht,
zunächst in einer ersten Stufe, gegebenenfalls in Gegenwart eines Verdünnungsmittels, wie beispielsweise Ethanol oder Toluol und gegebenenfalls in Gegenwart eines Reaktionshilfsmittels wie beispielsweise Schwefelsäure bei Temperaturen zwischen -30 °C und +150 °C umsetzt und die so erhältlichen Arylhydrazone der Formel (X),
R¹-CH=N-NH-Ar (X)
in welcher
R¹ und Ar die oben angegebene Bedeutung haben,
in einer 2. Stufe mit Halogenierungsmitteln wie beispielsweise N-Bromsuccinimid, N-Chlorsuccinimid oder Brom gegebenenfalls in Gegenwart eines Verdünnungsmittels wie beispielsweise Dimethylformamid oder Essigsäure bei Temperaturen zwischen -30 °C und +100 °C umsetzt.

Die N-Arylhydrazone der Formel (X) sind teilweise bekannt (vergl. z. B. Bull. Chem. Soc. Japan 58, 1841 (1985); J. Heterocyclic Chem. 22, 565 (1985); Chem. Lett. 1982, 543).

Die Aldehyde der Formel (VIII), bzw. deren Hydrate oder Halbacetale der Formel (IX) sind allgemein bekannte Verbindungen der organischen Chemie (vergl. z. B. J. Am. chem. Soc., 76, 300 [1954]).

Die Arylhydrazine der Formel (VII) sind bekannt (vergl. z. B. EP 154 115, EP 187 285, EP 34 945) oder erhältlich nach allgemein bekannten Verfahren in Analogie zu bekannten Verbindungen (vergl. z.B. Houben-Weyl, "Methoden der organischen Chemie", Band X, 2, Thieme Verlag, Stuttgart 1967).

Die zur Durchführung des erfindungsgemäßen Verfahrens (a) weiterhin als Ausgangsstoffe benötigten Isoxazole sind durch die Formel (III) allgemein definiert. In dieser Formel (III) steht R³ vorzugsweise für diejenigen Reste, die bereits im Zusammenhang mit der Beschreibung der erfindungsgemäßen Stoffe der Formel (I) als bevorzugt für diesen Substituenten genannt wurden.

Die Isoxazole der Formel (III) sind größtenteils allgemein bekannte Verbindungen der organischen Chemie (vergl. z. B. DE-OS 32 12 137, EP 91 022, Heterocycles 12, 1343 [1979]; Comprehensive Org. Chemistry 4, 993 [1979]).

Noch nicht bekannt sind Isoxazole der Formel (IIIa), 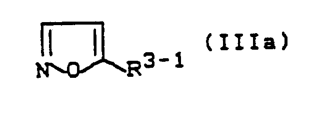 in welcher
R³⁻¹ für Fluoralkyl, insbesondere für Trifluormethyl, Dichlorfluormethyl oder Chlordifluormethyl steht.

Sie sind jedoch Gegenstand einer vorgängigen noch nicht veröffentlichten Patentanmeldung (vergl. Deutsche Patentanmeldung P 36 42 453 vom 12.12.1986).

Man erhält sie beispielsweise, wenn man Isoxazole der Formel (IIIb), 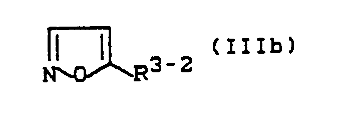 in welcher
R³⁻² für Chloralkyl, insbesondere für Trichlormethyl steht,
mit Fluorierungsmitteln wie beispielsweise Fluorwasserstoff oder Antimontrifluorid in der Gasphase bei Temperaturen zwischen 50 °C und 250 °C und bei einem Druck zwischen 1 und 50 bar umsetzt.

Chloralkyl-Isoxazole der Formel (IIIb) sind bekannt (vergl. z. B. Synthesis 1986, 69 - 70).

Die zur Durchführung des erfindungsgemäßen Verfahrens (b-α) weiterhin als Ausgangsstoffe benötigten Carbonylverbindungen sind durch die Formel (IV) allgemein definiert. In dieser Formel (IV) steht R³ vorzugsweise für diejenigen Reste, die bereits im Zusammenhang mit der Beschreibung der erfindungsgemäßen Stoffe der Formel (I) als bevorzugt für diesen Substituenten genannt wurden. R²⁻¹ steht vorzugsweise für diejenigen Reste, die bereits im Zusammenhang mit der Beschreibung der erfindungsgemäßen Stoffe der Formel (I) als bevorzugt für den Substituenten R² genannt wurden mit Ausnahme des Hydroxycarbonylrestes.

Die Carbonylverbindungen der Formel (IV) sind allgemein bekannte Verbindungen der organischen Chemie (vergl. z. B. Organic Reactions 1, 266 [1947]; Synthesis 1984, 1).

Die zur Durchführung des erfindungsgemäßen Verfahrens (b-β) weiterhin als Ausgangsstoffe benötigten Acrylnitrilderivate sind durch die Formel (V) allgemein definiert. In dieser Formel (V) steht R³ vorzugsweise für diejenigen Reste, die bereits im Zusammenhang mit der Beschreibung der erfindungsgemäßen Stoffe der Formel (I) als bevorzugt für diesen Substituenten genannt wurden. R²⁻¹ steht vorzugsweise für diejenigen Reste, die bereits im Zusammenhang mit der Beschreibung der erfindungsgemäßen Stoffe der Formel (I) als bevorzugt für den Substituenten R² genannt wurden mit Ausnahme des Hydroxycarbonylrestes.

Die Acrylnitrilderivate der Formel (V) sind allgemein bekannte Verbindungen der organischen Chemie.

Die zur Durchführung des erfindungsgemäßen Verfahrens (b-γ) weiterhin als Ausgangsstoffe benötigten Alkine sind durch die Formel (VI) allgemein definiert. In dieser Formel (VI) steht R³ vorzugsweise für diejenigen Reste, die bereits im Zusammenhang mit der Beschreibung der erfindungsgemäßen Stoffe der Formel (I) als bevorzugt für diesen Substituenten genannt wurden. R²⁻¹ steht vorzugsweise für diejenigen Reste, die bereits im Zusammenhang mit der Beschreibung der erfindungsgemäßen Stoffe der Formel (I) als bevorzugt für den Substituenten R² genannt wurden mit Ausnahme des Hydroxycarbonylrestes.

Die Alkine der Formel (VI) sind allgemein bekannte Verbindungen der organischen Chemie (vergl. z. B. Houben-Weyl "Methoden der organischen Chemie", Band V/2a, S. 1, Thieme Verlag Stuttgart 1977).

Die zur Durchführung des erfindungsgemäßen Verfahrens (c) als Ausgangsstoffe benötigten 3-Halogenalkyl-1-aryl-pyrazole sind durch die Formel (Id) allgemein definiert. In dieser Formel (Id) stehen R¹, R³ und Ar vorzugsweise für diejenigen Reste, die bereits im Zusammenhang mit der Beschreibung der erfindungsgemäßen Stoffe der Formel (I) als bevorzugt für diese Substituenten genannt wurden. R²⁻³ steht vorzugsweise für Cyano oder für geradkettiges oder verzweigtes Alkoxycarbonyl mit 1 bis 4 Kohlenstoffatomen, insbesondere für Cyano, Methoxycarbonyl oder Ethoxycarbonyl.

Die 3-Halogenalkyl-1-aryl-pyrazole der Formel (Id) sind erfindungsgemäße Verbindungen und erhältich mit Hilfe der erfindungsgemäßen Verfahren (a) oder (b).

Als Verdünnungsmittel zur Durchführung der erfindungsgemäßen Verfahren (a) und (b-α) kommen inerte organische Lösungsmittel infrage. Vorzugsweise verwendet man polare Lösungsmittel wie beispielsweise Dioxan, Tetrahydrofuran, Dimethylformamid, Ethanol, Methanol, t-Butanol oder Ethylenglykolmonomethylether.

Die erfindungsgemäßen Verfahren (a) und (b-α) werden vorzugsweise in Gegenwart eines geeigneten Säurebindemittels durchgeführt. Als solche kommen alle üblicher weise verwendbaren anorganischen oder organischen Basen infrage. Vorzugsweise verwendet man Alkalimetallhydride, -hydroxide oder -alkoholate, wie beispielsweise Natriumhydrid, Natriumhydroxid, Natriummethylat, Natriumethylat, Natriumisopropylat, Kaliumisopropylat oder Kalium-t-butylat.

Die Reaktionstemperaturen können bei der Durchführung der erfindungsgemäßen Verfahren (a) und (b-α) in einem größeren Bereich variiert werden. Im allgemeinen arbeitet man bei Temperaturen zwischen -30 °C und +150 °C, vorzugsweise bei Temperaturen zwischen 0 °C und +100 °C.

Zur Durchführung des erfindungsgemäßen Verfahrens (a) setzt man pro Mol an N-Arylhydrazidhalogenid der Formel (II) im allgemeinen 1,0 bis 10,0 Mol, vorzugsweise 1,0 bis 2,0 Mol an Isoxazole der Formel (III) und gegebenenfalls 1,0 bis 10,0 Mol, vorzugsweise 1,0 bis 2,0 Mol an Säurebindemittel ein. Die Reaktionsdurchführung, Aufarbeitung und Isolierung der Reaktionsprodukte der Formel (Ia) erfolgt nach allgemein bekannten Verfahren (vergl. auch die Herstellungsbeispiele).

Zur Durchführung des erfindungsgemäßen Verfahren (b-α) setzt man pro Mol an N-Arylhydrazid-halogenid der Formel (II) im allgemeinen 1.0 bis 10.0 Mol, vorzugsweise 1.0 bis 2.0 Mol an Carbonylverbindung der Formel (IV) und gegebenenfalls 1.0 bis 10.0 Mol, vorzugsweise 1.0 bis 2.0 Mol an Säurebindemittel ein. Die Reaktionsdurchführung, Aufarbeitung und Isolierung der Reaktionsprodukte der Formel (Ib) erfolgt nach allgemein bekannten Verfahren (vergl. auch die Herstellungsbeispiele).

Als Verdünnungsmittel zur Durchführung der erfindungsgemäßen Verfahren (b-β) und (b-γ) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Die erfindungsgemäßen Verfahren (b-β) und (b-γ) werden vorzugsweise in Gegenwart eines geeigneten Säurebindemittels durchgeführt. Als solche kommen alle üblichen anorganischen oder organischen Basen in Frage. Vorzugsweise verwendet man tertiäre Amine wie Triethylamin, N,N-Dimethylanilin, Pyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (b-β) und (b-γ) in einem größeren Bereich variiert werden. Im allgemeinen arbeitet man bei Temperaturen zwischen -30 °C und +150 °C, vorzugsweise bei Temperaturen zwischen 0 °C und 100 °C.

Zur Durchführung des erfindungsgemäßen Verfahrens (b-β) setzt man pro Mol an N-Arylhydrazid-halogenid der Formel (II) im allgemeinen 1.0 bis 20.0 Mol, vorzugsweise 1.0 bis 5.0 Mol an Acrylnitrilderivat der Formel (V) und gegebenenfalls 1.0 bis 20.0 Mol, vorzugsweise 1.0 bis 2.0 Mol an Säurebindemittel ein.
Die Reaktionsdurchführung, Aufarbeitung und Isolierung der Reaktionsprodukte erfolgt nach allgemein üblichen Methoden (vergl. auch die Herstellungsbeispiele).

Zur Durchführung des erfindungsgemäßen Verfahrens (b-γ) setzt man pro Mol an N-Arylhydrazid-halogenid der Formel (II) im allgemeinen 1.0 bis 20.0 Mol, vorzugsweise 1.0 bis 5.0 Mol an Alkin der Formel (VI) und gegebenenfalls 1.0 bis 20.0 Mol, vorzugsweise 1.0 bis 2.0 Mol an Säurebindemittel ein.
Die Reaktionsdurchführung, Aufarbeitung und Isolierung der Reaktionsprodukte erfolgt nach allgemein üblichen Methoden (vergl. auch die Herstellungsbeispiele).

Das erfindungsgemäße Verfahren (c) wird in Gegenwart einer geeigneten Säure durchgeführt. Als solche kommen alle üblicherweise verwendbaren anorganischen oder organischen Säuren infrage. Vorzugsweise verwendet man Schwefelsäure, Chlorwasserstoffsäure oder p-Toluolsulfonsäure.

Als Verdünnungsmittel zur Durchführung des erfindungsgemäßen Verfahrens (c) kommen polare anorganische oder organische Lösungsmittel oder deren Gemische mit Wasser in Frage. Vorzugsweise verwendet man die gleichzeitig als Reagenz eingesetzte Schwefelsäure in entsprechendem Überschuß als Verdünnungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und +150 °C, vorzugsweise bei Temperaturen zwischen +20 °C und +100 °C.

Zur Durchführung des erfindungsgemäßen Verfahrens (c) setzt man pro Mol an 3-Halogenalkyl-1-aryl-pyrazol der Formel (Id) im allgemeinen 1,0 bis 30 Mol, vorzugsweise 1,0 bis 10,0 Mol an wässriger Säure ein. Die Reaktionsdurchführung, Aufarbeitung und Isolierung der Reaktionsprodukte erfolgt nach allgemein bekannten Verfahren (vergl. auch die Herstellungsbeispiele).

Die Wirkstoffe eignen sich zur Bekämpfung von tierischen Schädlingen, vorzugsweise Arthropoden und insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelisbilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Dabei lassen sich die erfindungsgemäßen Wirkstoffe mit besonders gutem Erfolg zur Bekämpfung von Blatt- und Bodeninsekten, wie beispielsweise gegen die Larven der Meerrettichblattkäfer (Phaedon cochleariae), oder gegen die Maden der Zwiebelfliege (Phorbia antiqua) sowie gegen adulte Formen des Meerrettichblattkäfers (Phaedon cochleariae) einsetzen. Bei dieser Anwendung zeigen die erfindungsgemäßen Wirkstoffe neben einer guten protektiven Wirksamkeit auch hervorragende systemische Eigenschaften. Darüberhinaus lassen sich die erfindungsgemäßen Wirkstoffe auch sehr gut zur Bekämpfung von Hygiene- und Vorratsschädlingen, wie beispielsweise gegen die gemeine Stubenfliege (Musca domestica) oder gegen die Deutsche Hausschabe (Blattella germanica) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen, u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Triebgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farb stoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwen dungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

### Herstellungsbeispiele

### Beispiel 1

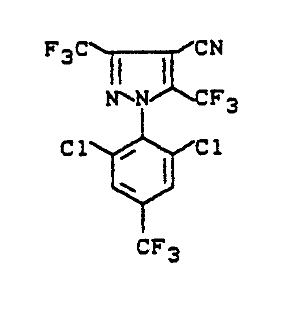

### (Verfahren a)

Man löst 0,92 g (0.04 Mol) Natrium in 25 ml Ethanol, gibt bei 0 °C 5,48 g (0.04 Mol) 5-Trifluormethylisoxazol zu und rührt 15 Minuten bei Raumtemperatur. Zu diesem Gemisch tropft man unter Kühlung 16,16 g (0.04 Mol) N-(2,6-Dichlor-4-trifluormethylphenyl)-trifluoracethydrazid-bromid und rührt noch 10 Stunden bei 25 °C nach. Nach Abfiltrieren des ausgefallenen Natriumbromids wird das Filtrat eingeengt und säulenchromatographisch aufgetrennt.

Man erhält 1,65 g (9,3 % der Theorie) 4-Cyan-3,5-di(trifluormethyl)-1-(2,6-dichlor-4-trifluormethylphenyl)-pyrazol vom Schmelzpunkt 88 °C - 92 °C.

### Herstellung der Ausgangsverbindung

### Beispiel III-1

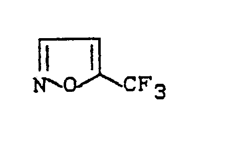

56 g (0.3 Mol) 5-Trichlormethyl-isoxazol (vergl. z. B. DE-OS 32 12 137), 80 g Antimontrifluorid und 1 ml Antimonpentachlorid werden bei Raumtemperatur zusammengefügt, dann auf eine Innentemperatur von 140 °C aufgeheizt und bei dieser Temperatur 90 Minuten unter Rückfluß reagieren gelassen. Anschließend werden die flüchtigen Anteile bei Normaldruck abdestilliert (70 °C bis 120 °C) und das erhaltene Rohprodukt über eine 10 cm-Füllkörperkolonne redestilliert.

Man erhält 26,8 g (65,2 % der Theorie) an 5-Trifluormethyl-isoxazol mit einem Siedepunkt von 79 °C bis 80 °C bei Normaldruck und einem Brechungsindex n$\frac{\text{20}}{\text{D}}$ = 1,3493.

### Beispiel 2

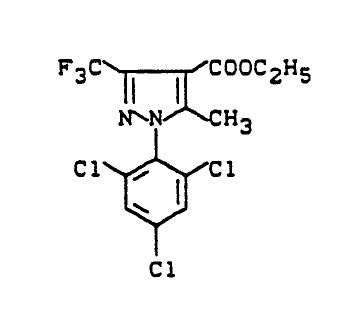

### (Verfahren b-α)

Eine Lösung von 2,72 g (0.04 Mol) Natriumethanolat in 20 ml Ethanol versetzt man mit 5,20 g (0.04 Mol) Acetessigsäureethylester und erhitzt 15 Minuten auf Rückflußtemperatur. Anschließend tropft man 14,8 g (0.04 Mol) N-(2,4,6-Trichlorphenyl)-trifluoracethydrazidbromid zu und kocht weitere 5 Stunden bei Rückflußtemperatur. Nach Abkühlen auf Raumtemperatur filtriert man ausgefallenes Natriumbromid ab und engt ein.

Säulenchromatographische Auftrennung des Rückstands ergibt 4,56 g (28 % der Theorie) 4-Ethoxycarbonyl-5-methyl-3-trifluormethyl-1-(2,4,6-trichlorphenyl)-pyrazol vom Schmelzpunkt 95 °C - 96 °C.

### Beispiel 3

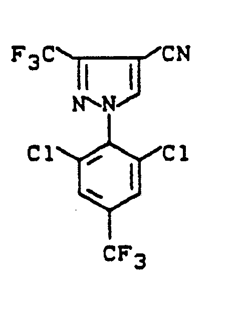

### Verfahren b-β)

12,6 g (0.125 Mol) Triethylamin tropft man bei 90 °C unter Rühren zu einem Gemisch aus 20,2 g (0.05 Mol) N-(2,6-Dichlor-4-trifluormethylphenyl)-trifluoracethydrazid-bromid, 7,8 g (0.10 Mol) Fumarsäuredinitril und 20 ml Toluol. Man rührt noch weitere 5 Stunden bei 90 °C, kühlt ab und engt ein.

Durch säulenchromatographische Auftrennung des Rückstands erhält man 2,79 g (15 % der Theorie) 4-Cyan-1-(2,6-dichlor-4-trifluormethylphenyl)-pyrazol vom Schmelzpunkt 116 °C - 118 °C.

### Beispiel 4

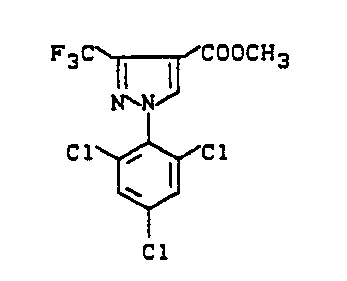

### (Verfahren b-γ)

Zu einem Gemisch aus 12,97 g (0.035 Mol) N-(2,4,6-Trichlorphenyl)-trifluoracethydrazid-bromid, 6,47 g (0.077 Mol) Propionsäuremethylester und 25 ml Toluol tropft man bei 75 °C 8,86 g (0.088 Mol) Triethylamin und rührt noch weitere 2 Stunden bei 75 °C nach. Nach Abkühlen auf Raumtemperatur filtriert man vom Natriumbromid ab und engt ein.

Durch Säulenchromatographie erhält man 5,79 g (44,3 % der Theorie) 4-Methoxycarbonyl-3-trifluormethyl-1-(2,4,6-trichlorphenyl)-pyrazol vom Schmelzpunkt 107 °C - 108 °C.

### Beispiel 5

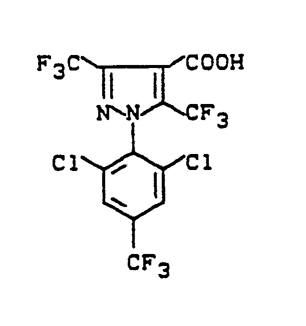

### (Verfahren c)

Ein Gemisch aus 0,98 g (0.002 Mol) 4-Ethoxycarbonyl-3,5-di(trifluormethyl)-1-(2,6-dichlor-4-trifluormethylphenyl)-pyrazol und 4 ml konz. Schwefelsäure wird 5 Stunden auf 75 °C erhitzt und anschließend auf Eis gegossen.

Nach Absaugen erhält man 0,79 g (86 % der Theorie) 4-Hydroxycarbonyl-3,5-di(trifluormethyl)-1-(2,6-dichlor-4-trifluormethylphenyl)-pyrazol vom Schmelzpunkt 185 °C - 190 °C.

### Beispiel 6

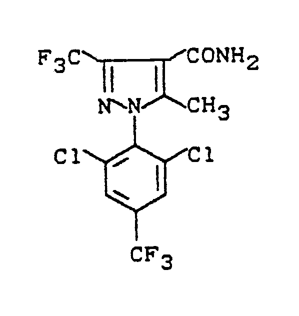

### (Verfahren c)

0,97 g (0,0025 Mol) 4-Cyan-5-methyl-3-trifluormethyl-1-(2,6-dichlor-4-trifluormethylphenyl)-pyrazol in 4 ml konz. Schwefelsäure werden 5 Stunden auf 75 °C erhitzt. Nach Abkühlen auf Raumtemperatur verdünnt man mit Eiswasser und saugt ab.

Man erhält 0,70 g (69 % der Theorie) 4-Aminocarbonyl-5-methyl-3-trifluormethyl-1-(2,6-dichlor-4-trifluormethylphenyl)-pyrazol vom Schmelzpunkt 165 °C - 170 °C.

In entsprechender Weise und gemäß den allgemeinen Angaben zur Herstellung erhält man die in der folgenden Tabelle aufgeführten 3-Halogenalkyl-1-aryl-pyrazole der allgemeinen Formel (I):

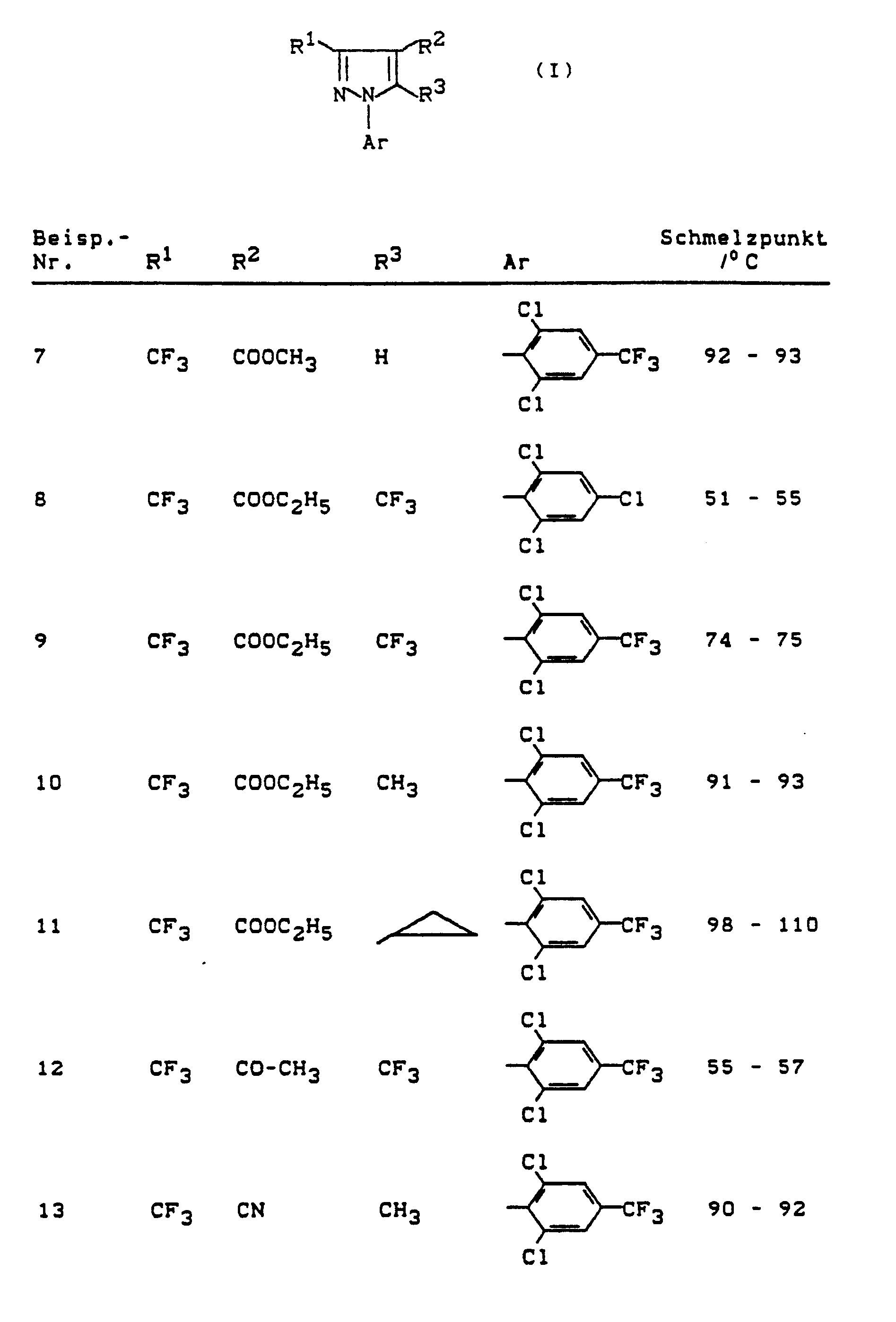

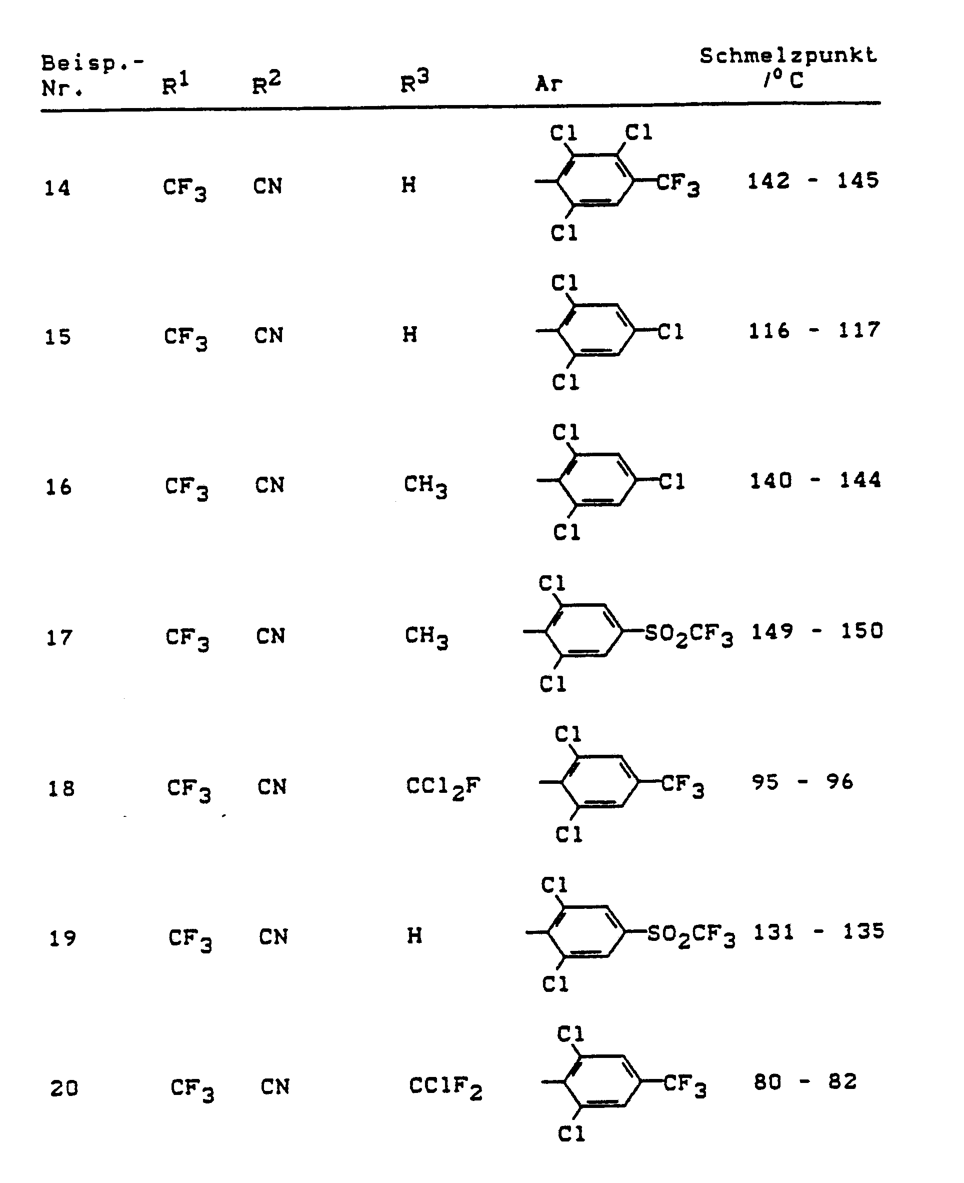

### Herstellung der Ausgangsverbindungen

### Beispiel II-1

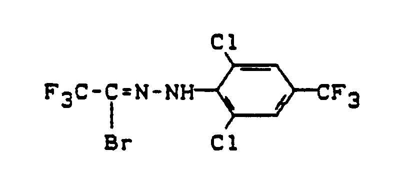

Zu einer Lösung von 8,12 g (0.025 Mol) Trifluoracetaldehyd-N-(2,6-dichlor-4-trifluormethylphenyl)-hydrazon in 7,5 ml Dimethylformamid gibt man bei Raumtemperatur portionsweise 4,45 g (0.025 Mol) N-Bromsuccinimid, wobei eine exotherme Reaktion auftritt. Man rührt 3 Stunden bei Raumtemperatur, destilliert das Dimethylformamid ab und versetzt den Rückstand mit 20 ml Petrolether. Nach Absaugen des ausgefallenen Succinimids wird das Filtrat eingeengt und einer Kugelrohrdestillation unterworfen.

Man erhält 9,26 g (91,7 % der Theorie) N-(2,6-Dichlor-4-trifluormethylphenyl)-trifluoracethydrazid-bromid vom Siedepunkt 110 °C bei 0,06 mbar und vom Brechungsindex n$\frac{\text{20}}{\text{D}}$ 1,510.

### Beispiel II-2

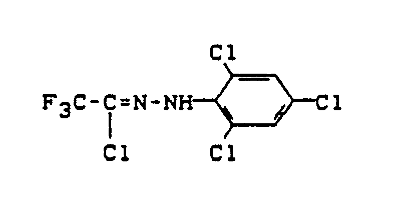

3,2 g (0.011 Mol) Trifluoracetaldehyd N-(2,4,6-Trichlorphenyl)-hydrazon und 1,5 g (0.011 Mol) N-Chlorsuccinimid in 3,3 ml Dimethylformamid werden 3 Stunden bei Raumtemperatur gerührt. Nach Einengen versetzt man mit Petrolether, filtriert und engt wieder ein.

Kugelrohrdestillation des Rückstands bei 150 °C (0,5 mbar) liefert 3,28 g (89,6 % der Theorie) N-(2,4,6-Trichlorphenyl)-trifluoracethydrazid-chlorid vom Brechungsindex n$\frac{\text{20}}{\text{D}}$ 1,549.

In entsprechender Weise und gemäß den allgemeinen Angaben zur Herstellung erhält man die folgenden N-Arylhydrazid-halogenide der allgemeinen Formel (II):
R¹ 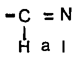 -NH-Ar (II)

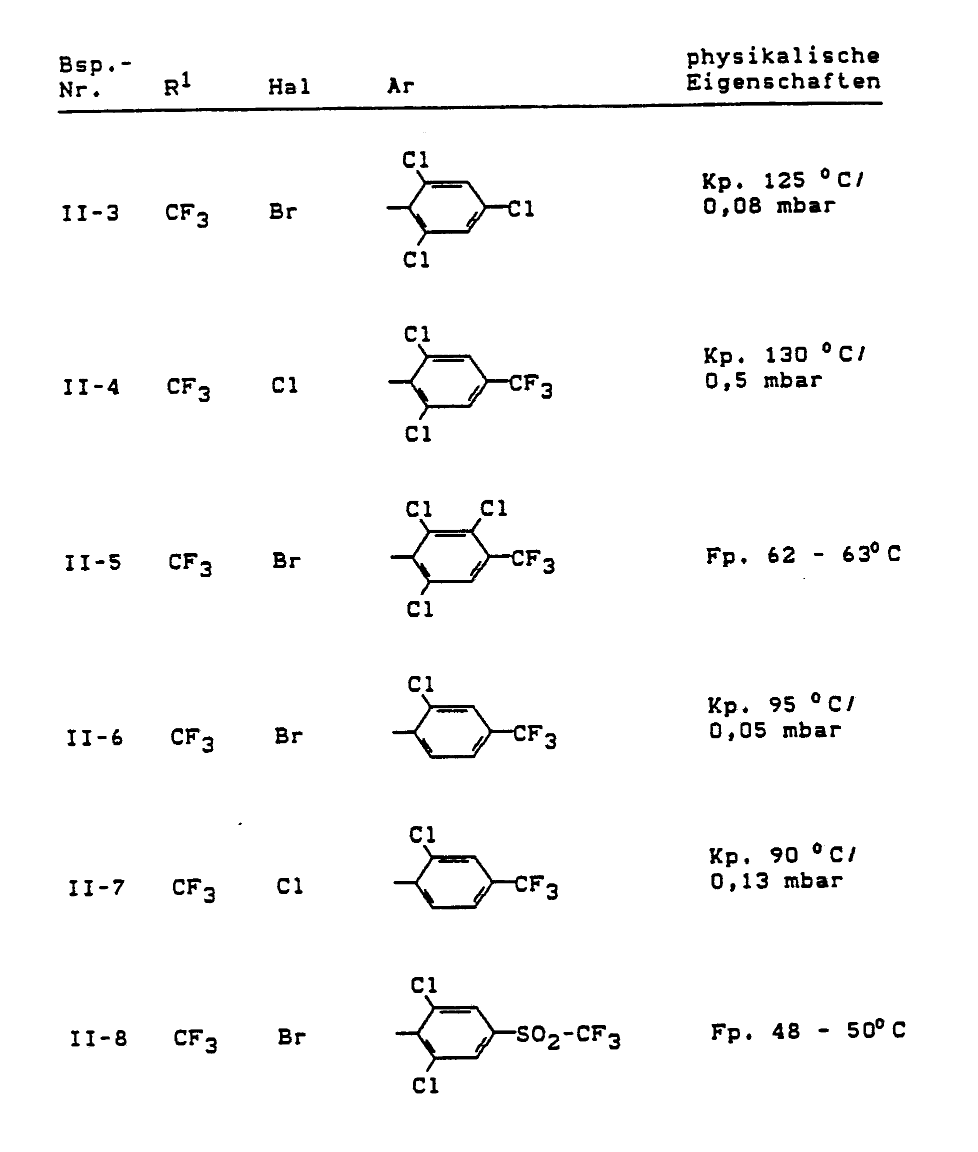

### Beispiel X-1

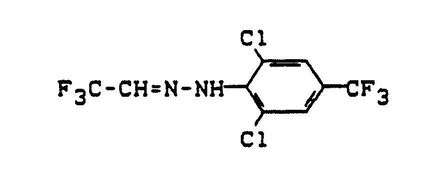

Ein Gemisch von 100 g (0.69 Mol) Trifluoracetaldehydethylhalbacetal und 169,1 g (0.69 Mol) 2,6-Dichlor-4-trifluormethylphenylhydrazin wird 6 Stunden bei 100 °C erhitzt. Nach Entfernung der flüchtigen Komponente wird der Rückstand aus Petrolether umkristallisiert.

Man isoliert 200 g (89 % der Theorie) Trifluoracetaldehyd-N-(2,6-dichlor-4-trifluormethylphenyl)-hydrazon vom Schmelzpunkt 45 °C bis 46 °C.

In entsprechender Weise und gemäß den allgemeinen Angaben zur Herstellung erhält man die folgenden Aldehyd-N-arylhydrazone der allgemeinen Formel (X):
R¹-CH=N-NH-Ar (X)

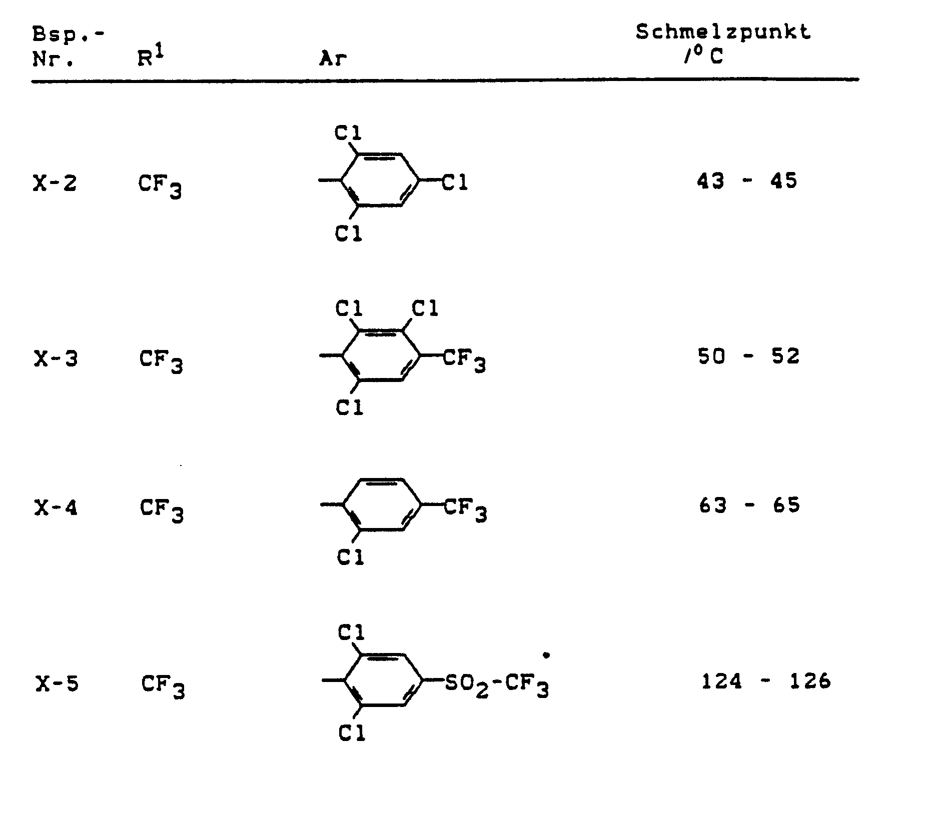

### Anwendungsbeispiele

In den folgenden Anwendungsbeispielen wurde die nachstehend aufgeführte Verbindung als Vergleichsubstanz eingesetzt:

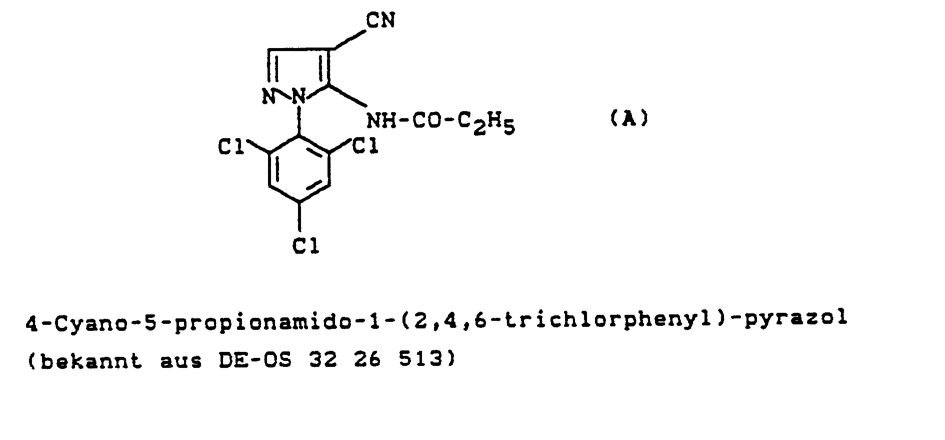

### Beispiel A

### Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegen- über dem Stand der Technik; 3, 7, 13, 15, 17, 18, 19, 20.

### Beispiel B

### Saatgutbehandlungs-Test/Wurzelsystemische Wirkung

Testinsekt: Phaedon cochleariae-Käfer
Testpflanze: Brassica oleracea
Lösungsmittel: 1 Gewichtsteil Aceton
Trägermaterial: Kaolin

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Kohl-Saatgut wird mit dieser Wirkstoffzubereitung mit den geforderten Aufwandmengen behandelt. Die Kohl-Aussaat erfolgt in 0,5-l-Töpfen mit standardisierten Böden bei 20 °C Raumtemperatur.

Der Wirkstoff kann so von den Pflanzenwurzeln aus dem Boden aufgenommen und in die Blätter transportiert werden.

Für den Nachweis des wurzelsystemischen Effektes werden nach 14 Tagen ausschließlich die Blätter mit den obengenannten Testtieren besetzt. Nach weiteren 3 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 3.

### Beispiel C

### Saatgutbehandlungs-Test/Bodeninsekten

Testinsekt: Phorbia antiqua-Maden im Boden
Testpflanze: Allium cepa
Lösungsmittel: 1 Gewichtsteil Aceton
Trägermaterial: Kaolin

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Zwiebelsaatgut wird mit dieser Wirkstoffzubereitung mit den geforderten Aufwandmengen behandelt. Die Aussaat erfolgt in 0,5-l-Töpfen mit standardisierten Böden bei 20 °C Gewächshaustemperatur.

Nach Auflauf der Zwiebeln werden diese mit Zwiebelfliegeneiern künstlich infiziert.

Die Auswertung erfolgt nach 14 Tagen. Der Wirkungsgrad ist 100 %, wenn alle Zwiebelpflanzen stehenbleiben, 0 %, wenn alle Testpflanzen (wie in der unbehandelten Kontrolle) vernichtet wurden.

Bei diesem Test zeigen z. B. die folgenden Verbindungen der Herstellungsbeispiele überlegene Wirkung gegenüber dem Stand der Technik: 3.

### Beispiel D

### LT₁₀₀-Test für Dipteren

Testtiere: Musca domestica (resistent)
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m² Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z. B. die folgenden Verbindungen der Herstellungsbeispiele überlegene Wirkung gegenüber dem Stand der Technik: 1, 3 und 13.

### Beispiel E

### LD₁₀₀-Test

Testtiere: Blattella germanica
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m² Filterpapier verschieden hoch. Anschließend gibt man etwa 20 der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z. B. die folgenden Verbindungen der Herstellungsbeispiele überlegene Wirkung gegenüber dem Stand der Technik: 1, 3 und 13.

## Patentansprüche

1. 3-Halogenalkyl-1-aryl-pyrazole der allgemeinen Formel (I), 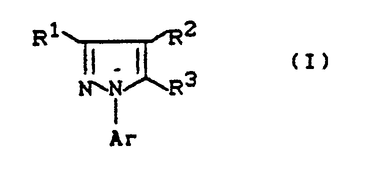 in welcher
R¹ für Halogenalkyl steht,
R² für Cyano, für Hydroxycarbonyl, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkylcarbonyl, Halogenalkylcarbonyl oder Cycloalkylcarbonyl steht,
R³ für Wasserstoff, Alkyl, Halogenalkyl oder Cycloalkyl steht und
Ar für substituiertes Phenyl mit Ausnahme des 2,4-Dinitrophenylrestes steht.

2. 3-Halogenalkyl-1-aryl-pyrazole gemäß Anspruch 1, Formel (I), in welcher
R¹ für geradkettiges oder verzweigtes Halogenalkyl mit 1 bis 4 Kohlenstoffatomen und 1 bis 9 gleichen oder verschiedenen Halogenatomen steht,
R² für Cyano, für Hydroxycarbonyl, für Carbamoyl, Thiocarbamoyl, für jeweils geradkettiges oder verzweigtes Alkoxycarbonyl, N-Alkylcarbamoyl oder N,N-Dialkylcarbamoyl mit jeweils 1 bis 4 Kohlenstoffatomen in den einzelnen Alkylteilen, für jeweils geradkettiges oder verzweigtes Alkenyloxycarbonyl, N-Alkenylcarbamoyl oder N,N-Dialkenylcarbamoyl mit jeweils 3 bis 6 Kohlenstoffatomen in den einzelnen Alkenylteilen, für jeweils geradkettiges oder verzweigtes Alkinyloxycarbonyl, N-Alkinylcarbamoyl oder N,N-Dialkinylcarbamoyl mit jeweils 3 bis 6 Kohlenstoffatomen in den einzelnen Alkinylteilen, oder für gegebenenfalls im Phenylteil einfach oder mehrfach, gleich oder verschieden substituiertes Phenylcarbamoyl steht, wobei als Substituenten in Frage kommen: Halogen, Cyano, Nitro sowie jeweils geradkettiges oder verzweigts Alkyl, Alkoxy, Alkylthio, Halogenalkyl, Halogenalkoxy oder Halogenalkylthio mit jeweils 1 bis 4 Kohlenstoffatomen und im Fall des Halogenalkyl, Halogenalkoxy oder Halogenalkylthio mit 1 bis 9 gleichen oder verschiedenen Halogenatomen; außerdem für jeweils geradkettiges oder ver zweigtes Alkylcarbonyl oder Halogenalkylcarbonyl mit jeweils 1 bis 5 Kohlenstoffatomen und im Fall des Halogenalkylcarbonyl mit 1 bis 9 gleichen oder verschiedenen Halogenatomen oder für Cycloalkylcarbonyl mit 4 bis 8 Kohlenstoffatomen steht,
R³ für Wasserstoff, für jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen oder für Cycloalkyl mit 3 bis 7 Kohlenstoffatomen steht und
Ar für einfach oder mehrfach, gleich oder verschieden substituiertes Phenyl mit Ausnahme des 2,4-Dinitrophenylrestes steht, wobei als Substituenten infrage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenenfalls 1 bis 9 gleichen oder verschiedenen Halogenatomen.

3. 3-Halogenalkyl-1-aryl-pyrazole gemäß Anspruch 1, Formel (I), in welcher
R¹ für Trifluormethyl, Dichlorfluormethyl, Difluorchlormethyl, Difluormethyl, Trifluorethyl, Trifluorchlorethyl, Trifluordichlorethyl, Difluordichlorethyl, Tetrafluorethyl, Pentafluorethyl, Heptafluorpropyl oder Nonafluorbutyl steht,
R² für Cyano, für Hydroxycarbonyl, für Carbamoyl, Thiocarbamoyl, für Methoxycarbonyl, Ethoxycarbonyl, N-Methylcarbamoyl, N,N-Dimethylcarbamoyl, N-Ethylcarbamoyl, N,N-Diethylcarbamoyl, für Acetyl, Propionyl, Trifluoracetyl, Dichlorfluoracetyl, Difluorchloracetyl, für Cyclopropylcarbonyl, Cyclohexylcarbonyl oder für im Phenylteil gegebenenfalls ein- bis dreifach gleich oder verschieden durch Fluor, Chlor, Brom, Cyano, Nitro, Methyl, Ethyl, n- oder i-Propyl, Methoxy, Ethoxy, n- oder i-Propoxy, Methylthio, Trifluormethyl, Trifluormethoxy oder Trifluormethylthio substituiertes N-Phenylcarbamoyl steht,
R³ für Wasserstoff, Methyl, Ethyl, n- oder i-Propyl, n-, i-, s- oder t-Butyl, Trifluormethyl, Dichlorfluormethyl, Difluorchlormethyl, Difluormethyl, Trifluorethyl, Tetrafluorethyl, Pentafluorethyl, Trifluorchlorethyl, Trifluordichlorethyl, Difluordichlorethyl, Heptafluorpropyl, Nonafluorbutyl, für Cyclopropyl, Cyclobutyl, Cyclopentyl oder Cyclohexyl steht und
Ar für ein- bis fünffach, gleich oder verschieden substituiertes Phenyl mit Ausnahme des 2,4-Dinitro-phenylrestes steht, wobei als Substituenten infrage kommen: Fluor, Chlor, Brom, Iod, Cyano, Nitro, Methyl, Ethyl, n-oder i-Propyl, n-, i-, s- oder t-Butyl, Methoxy, Ethoxy, Methylthio, Methylsulfinyl, Methylsulfonyl, Methoxycarbonyl, Ethoxycarbonyl, Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl, Trifluordichlorethyl oder ein Rest -X-R⁴, wobei X für Sauerstoff, Schwefel, Sulfinyl oder Sulfonyl steht und R⁴ für Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl oder Trifluordichlorethyl steht.

4. Verfahren zur Herstellung von 3-Halogenalkyl-1-aryl-pyrazolen der allgemeinen Formel (I), 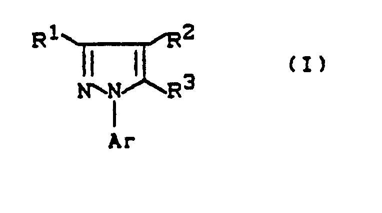 in welcher
R¹ für Halogenalkyl steht,
R² für Cyano, für Hydroxycarbonyl, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkylcarbonyl, Halogenalkylcarbonyl oder Cycloalkylcarbonyl steht,
R³ für Wasserstoff, Alkyl, Halogenalkyl oder Cycloalkyl steht und
Ar für substituiertes Phenyl mit Ausnahme des 2,4-Dinitrophenylrestes steht,
dadurch gekennzeichnet,
(a) daß man zum Erhalt der 3-Halogenalkyl-1-aryl-pyrazole der Formel (Ia), 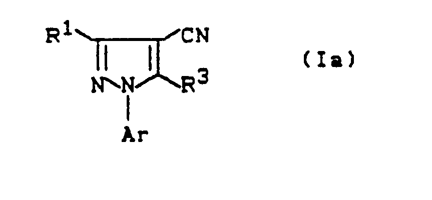 in welcher
R¹, R³ und Ar die oben angegebene Bedeutung haben,
N-Aryl-hydrazidhalogenide der Formel (II),
R¹- 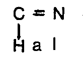 -NH-Ar (II)
in welcher
Hal für Halogen steht und
R¹ und Ar die oben angegebene Bedeutung haben,
mit Isoxazolen der Formel (III), 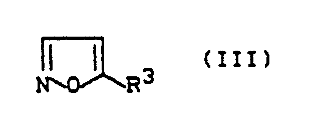 in welcher
R³ die oben angegebene Bedeutung hat,
gegebenenfalls in Gegenwart eines Verdünnungsmittels und gegebenenfalls in Gegenwart eines Säurebindemittels umsetzt;
(b) oder daß man zum Erhalt der 3-Halogenalkyl-1-aryl-pyrazole der Formel (Ib), 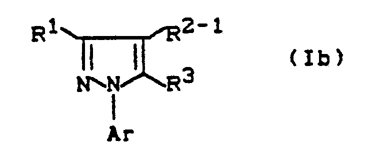 in welcher
R²⁻¹ für Cyano, für Carbamoyl, Thiocarbamoyl, für Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl, N-Arylcarbamoyl, für Alkylcarbonyl, Halogenalkylcarbonyl oder Cycloalkylcarbonyl steht und
R¹, R³ und Ar die oben angegebene Bedeutung haben,
N-Aryl-hydrazidhalogenide der Formel (II),
R¹- 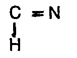 -NH-Ar (II)
in welcher
Hal für Halogen steht und
R¹ und Ar die oben angegebene Bedeutung haben,
entweder
(α) mit Carbonylverbindungen der Formel (IV),
R²⁻¹-CH₂- 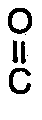 -R³ (IV)
in welcher
R²⁻¹ und R³ die oben angegebene Bedeutung haben,
oder
(β) mit Acrylnitrilderivaten der Formel (V), 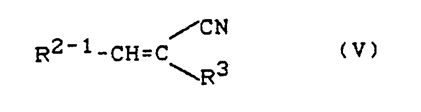 in welcher
R²⁻¹ und R³ die oben angegebene Bedeutungen haben,
oder
(γ) mit Alkinen der Formel (VI),
R²⁻¹-C≡C-R³ (VI)
in welcher
R²⁻¹ und R³ die oben angegebene Bedeutung haben,
jeweils gegebenenfalls in Gegenwart eines Verdünnungsmittels und gegebenenfalls in Gegenwart eines Säurebindemittels umsetzt;
(c) oder daß man zum Erhalt der 3-Halogenalkyl-1-aryl-pyrazole der Formel (Ic), 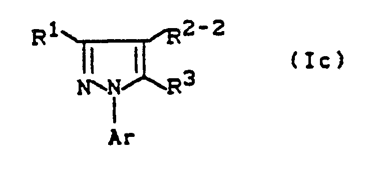 in welcher
R¹, R³ und Ar die oben angegebene Bedeutung haben und
R²⁻² für Hydroxycarbonyl oder Carbamoyl steht,
die mit Hilfe der Verfahren (a) oder (b) erhältlichen 3-Halogenalkyl-1-aryl-pyrazole der Formel (Id) 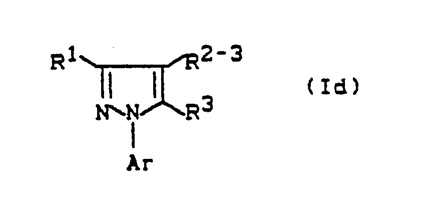 in welcher
R¹, R³ und Ar die oben angegebene Bedeutung haben und
R²⁻³ für Cyano, Alkoxycarbonyl, Alkenyloxycarbonyl oder Alkinyl steht,
mit Säuren gegebenenfalls in Gegenwart eines Verdünnungsmittels am Substituenten in 4-Position des Pyrazolringes verseift.

5. Insektizide Mittel, gekennzeichnet durch einen Gehalt an mindestens einem 3-Halogenalkyl-1-aryl-pyrazol der Formel (I).

6. Verfahren zur Bekämpfung von Insekten, dadurch gekennzeichnet, daß man 3-Halogenalkyl-1-aryl-pyrazole der Formel (I) auf Insekten und/oder ihren Lebensraum einwirken läßt.

7. Verwendung von 3-Halogenalkyl-1-aryl-pyrazolen der Formel (I) zur Bekämpfung von Insekten.

8. Verfahren zur Herstellung von insektiziden Mitteln, dadurch gekennzeichnet, daß man 3-Halogenalkyl-1-aryl-pyrazole der Formel (I) mit Streckmitteln und/oder oberflächenaktiven Mitteln vermischt.
